# NOUVEAU FASCICULE DE BREVET EUROPEEN

(11) **EP 0 459 856 B2**
(45) Date de publication et mention de la décision concernant l'opposition: **27.08.1997**
(45) Mention de la délivrance du brevet: 21.12.1994
(21) Numéro de dépôt: 91401252.1
(22) Date de dépôt: 15.05.1991
(51) Int. Cl.: A61K 7/06, A61K 7/08

(54) **Composition cosmétique ou pharmaceutique pour le traitement des cheveux et du cuir chevelu**
Kosmetische oder pharmazeutische Zubereitung zur Haar- und Kopfhautbehandlung
Cosmetic or pharmaceutical composition for hair and scalp treatment

(30) Priorité: 31.05.1990 FR 9006777
(43) Date de publication de la demande: 04.12.1991
(73) Titulaire: L'OREAL, 75008 Paris (FR)
(72) Inventeur: Grollier, Jean-François, F-75004 Paris (FR); Hansenne-Richoux, Isabelle, F-75017 Paris (FR)
(74) Mandataire: Peuscet, Jacques

(56) Documents cités:
- EP-A- 312 343
- EP-A- 0 373 988
- WO-A-87/06499
- DE-A- 3 447 621
- FR-A- 2 597 367
- GB-A- 2 157 168
- Cosmetics & Toiletries , Vol.100, 91 (1985)

## Description

La présente invention concerne une composition cosmétique ou pharmaceutique pour le traitement des cheveux et du cuir chevelu.

Il est bien connu que les cheveux sont sensibilisés ou fragilisés à des degrés divers par l'action des agents atmosphériques ainsi que par l'action de différents traitements cosmétiques, tels que permanentes, teintures ou décolorations. Les cheveux deviennent alors difficiles à démêler et à coiffer. De plus, ils deviennent rêches au toucher.

On a donc cherché des compositions, qui permettent de faciliter le démêlage et le coiffage des cheveux et améliorent leur douceur au toucher. Dans ce but, on utilise couramment des agents tensio-actifs cationiques. Lesdits agents tensio-actifs améliorent le démêlage et le coiffage mais présentent des inconvénients : ils ont tendance à alourdir la chevelure et à lui donner un aspect gras. Ces inconvénients sont d'autant plus accentués que les cheveux traités sont plus fins.

Dans le même but, on a également proposé d'utiliser des protéines quaternisées. Lesdites protéines ne présentent ni l'inconvénient d'alourdir les cheveux, ni celui de leur donner un aspect gras ; par contre, l'amélioration du démêlage et du coiffage des cheveux, qu'elles apportent, est nettement intérieure à celle apportée par les agents tensio-actifs cationiques.

On a donc essayé d'utiliser des compositions contenant à la fois un agent tensio-actif cationique et une protéine quaternisée. Mais, dans ce cas, l'effet obtenu est inférieur à la somme des effets que l'on pourrait obtenir séparément à l'aide de l'agent tensio-actif cationique et à l'aide de la protéine quaternisée car, dans la majorité des cas, la dépôt de l'agent tensio-actif cationique sur le cheveu freine celui de la protéine quaternisée. On a déjà proposé, dans GB-A-2 157 168, de remédier à cette situation par addition d'un polymère silicone cationique, qui améliore de façon sensible l'effet obtenu sur le cheveu.

Par ailleurs, il est connu, de très longue date, d'utiliser des huiles et des corps gras pour redonner aux cheveux de la douceur et du brillant ; l'application de ces composés est généralement suivie d'un shampooing pour éliminer du cheveu l'excédent d'huile ou de corps gras. Cependant, l'utilisation des huiles et corps gras amollit et alourdit les cheveux et il est impossible d'obtenir par la suite une coiffure ayant de la tenue et du volume.

Dans EP-A 0 373 988 qui n'est pas compris dans l'état de la technique au sens des Articles 54(2) et 89 CBE, on décrit une composition pour le traitement des cheveux et du cuir chevelu obtenue en associant à une phase aqueuse contenant un agent tensioactif cationique et/ou une protéine quaternisée, des lipides amphiphiles non-ioniques susceptibles de former une phase lamellaire lipidique hydratée.

Selon la présente invention, on a trouvé qu'en associant, à une solution aqueuse contenant au moins un agent tensio-actif cationique particulier et/ou une protéine quaternisée, des lipides ioniques susceptibles de former une phase lamellaire lipidique hydratée insoluble dans l'eau, on obtenait des compositions, qui permettent de démêler et coiffer facilement les cheveux sans les ramollir, ni les alourdir, ni les graisser. De plus, lesdites compositions confèrent aux cheveux des propriétés surprenantes quant au gonflant et permettent un gainage des cheveux de la racine à la pointe. Les cheveux traités ont donc du brillant et de la douceur et, après démêlage, ils sont lisses et légers de la racine à la pointe, même dans le cas des cheveux sensibilisés et des cheveux fins. Ces effets sont comparables à ceux définis par GB-A-2 157 168.

Mais on a également trouvé que les compositions selon la présente invention présentent, de façon surprenante, un effet hydratant sur le cuir chevelu. Elles ont l'avantage de procurer une sensation agréable de fraîcheur et de confort sur le cuir chevelu. Elles ont donc une double action : sur les cheveux et sur le cuir chevelu ; ce résultat est tout à fait nouveau et inattendu.

De plus, les compositions selon l'invention ont une excellente stabilité au stockage.

La présente invention a donc pour objet une composition cosmétique ou pharmaceutique pour le traitement des cheveux et du cuir chevelu renfermant des vesicules constitués d'une phase lamellaire hydratée de lipides amphiphiles ioniques, insolubles dans l'eau, éventuellement associés à un stabilisant, lesdites vesicules etant sous forme dispersée dans une phase aqueuse continue,caractérisée par le fait que ladite phase aqueuse contient :
1) au moins un agent tensio-actif cationique de formule : 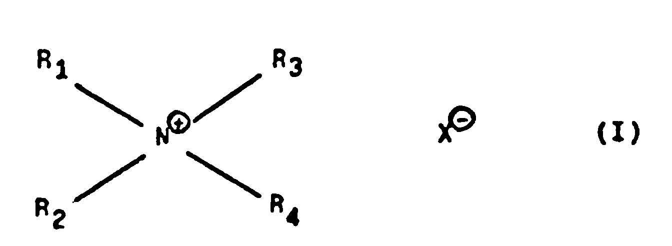 formule dans laquelle X est notamment le chlore ou CH₃ SO-₄ et R₁ est un radical alkyle en C₁- C₄, de préférence le radical méthyle, et dans laquelle :
   a) lorsque X est le chlore :
      - ou bien R₂ et R₃ sont des radicaux alkyle en C₁ - C₄ , identiques ou différents de R₁ et entre eux, et R₄ est un radical alkyle en C₁₆ - C₂₂;
      - ou bien R₂ = R₁ et, dans ce cas :
         - ou R₃ = R₄ = radical alkyle en C₁₈ ;
         - ou R₃ = radical (alkyle C₁₇ )amidopropyle et R₄ = radical (alkyle C₁₄)acétate ;
   b) lorsque X est CH₃SO⁻₄ :
      - R₂ désigne un radical (alkyle et/ou alkényl)amidoéthyle, dans lequel le radical alkyle et/ou alkényle est en C₁₃ - C₂₁ et dérive des acides gras du suif ;
      - R₃ et R₄ forment ensemble avec l'azote un hétérocycle 4-5-dihydroimidazole substitué, notamment un radical 2-(alkyle C₁₃ - C₂₁ dérivé des acides gras du suif)4,5-dihydroimidazole
      et/ou
2) au moins une protéine quaternisée constituée par un polypeptide modifié chimiquement portant, en bout de chaîne ou greffé sur celle-ci au moins un groupement ammonium quaternaire qui contient au moins un groupe alkyle en C₁-C₁₈, le polypeptide étant choisi parmi les hydrolysats de protéine animale ;
   sous réserve que si la composition contient comme agent tensio-actif cationique le chlorure de distéaryldiméthylammonium, ladite composition contienne obligatoirement au moins une protéine quaternisée telle que définie sous 2) ci-dessus, ledit (lesdits) agent(s) tensioactif(s) cationique(s), lorsqu'il(s) est (sont) présent(s), représentant en poids de 0,05 à 10 % du poids total de la composition, ladite (lesdites) protéine(s) quaternisée(s), lorsqu'elle(s) est (sont) présente(s), représentant en poids de 0.05 à 3 % du poids total de la composition et le(s) lipide(s) amphiphile(s) qui constitue(nt) la phase lamellaire représentant de 0,1 à 20 % du poids total de la composition.

Lorsque celle phase lamellaire lipidique hydratée forme des vésicules, celles-ci sont appelées des liposomes. Les liposomes contenus dans la composition selon l'invention ont avantageusement un diamètre moyen compris entre 0,01 et 5 µm, de préférence entre 0,05 et 0,35 µm.

Les liposomes sont bien connus dans l'état de la technique. Ce sont des sphérules ou vésicules constituées d'une ou plusieurs couches concentriques de lipides séparées par des couches de phase aqueuse interne. Dans le cas des liposomes, les lipides utilisées pour la fabrication des sphérules ou vésicules sont, de façon connue, des amphiphiles ioniques, d'origine naturelle ou synthétique, comportant, par molécule, une ou plusieurs longue(s) chaîne(s) hydrocarbonée(s).

Selon l'invention, le(s) composé(s) lipidique(s) amphiphile(s) ionique(s) constituant la phase lamellaire lipidique hydratée, insoluble dans l'eau, sont avantageusement choisis parmi les composés suivants :
a) les phospholipides naturels ou synthétiques , notamment la lécithine d'oeuf ou de soja, la sphingomyéline, la dipalmitoyl-phosphatidylcholine ou la lécithine hydrogénée ;
b) les composés amphotères comportant deux chaînes lipophiles ou une association de deux ions organiques à longue chaîne de signes opposés ;
c) les composés anioniques.

Parmi les composés anioniques, on choisit avantageusement ceux de formule : 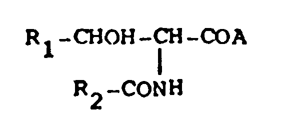 formule dans laquelle :
- R₁ désigne un radical alcoyle ou alcényle en C₇-C₂₁;
- R₂ désigne un radical hydrocarboné, saturé ou insaturé, en C₇-C₃₁ ;
- COA désigne un groupement choisi parmi les groupements suivants :
   - COOM, M étant H, Na, K, NH₄ ou un ion ammonium substitué derivé d'une amine ;
   - un reste 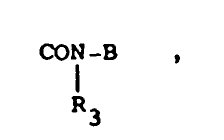 B étant un radical dérivé d'amines primaires ou secondaires, mono- ou polyhydroxylées et R₃ désignant un atome d'hydrogène ou un radical méthyle, éthyle ou hydroxyéthyle ;
   - un reste 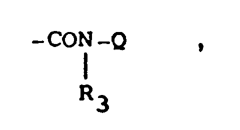 Q désignant un radical amino-alcoyle ou ammonioalcoyle substitué et R₃ ayant la signification indiquée ci-dessus ; et
- COOZ, Z représentant le reste d'un polyol en C₃-C₇.

De façon connue, ces composés lipidiques ioniques constituant la phase lamellaire lipidique hydratée peuvent être associés à au moins un additif stabilisant destiné à modifier la perméabilité ou la charge superficielle des feuillets lipidiques de la phase lamellaire lipidique hydratée. Selon l'invention, ces additifs sont plus particulièrement choisis dans le groupe formé par les stérols, tels que le cholestérol ou le bêta-sitostérol ; le sulfate de cholestérol (voir FR-A 2 597 367), les sels monosodiques ou disodiques de glutamates d'acyle, le radical acyle étant en C₁₄- C₂₂, tel que le sel monosodique du glutamate de stéaroyle, les sels disodiques de glutamates de cocoyle, de stéaroyle ou de mélanges de radicaux acylés dérivés du coprah et du suif, des esters phosphorique d'alcools gras en C₁₂-C₁₆ et des tensio-actifs lipophiles, tels que des phytostérols oxyéthylénés.

Les stabilisants anioniques sont associés aux composés lipidiques amphiphiles ioniques en une quantité ne dépassant, de préférence, pas 12 % en poids par rapport au poids du (ou des) lipide(s) amphiphile(s) ionique(s) constituant la phase lamellaire lipidique hydratée. Pour les stérols, et notamment le cholestérol, cette même proportion doit rester inférieure ou égale à 100 % en poids.

Selon l'invention, la phase lamellaire lipidique hydratée contient de l'eau en présence ou non d'un agent cosmétiquement ou pharmaceutiquement actif. A titre d'agent actif, on peut citer, par exemple, les agents anti-chute ou de repousse des cheveux, les rétinoîdes et apparentés, les anti-inflammatoires, les anti-fongiques, les anti-séborrhéiques, les filtres solaires, et analogues.

L'agent tensio-actif cationique contenu, selon l'invention, dans la phase aqueuse continue de la composition, est avantageusement choisi dans le groupe formé par :
a) les halogénures de tétraalkylammonium tels que le chlorure de béhényltriméthylammonium, le chlorure de distéaryldiméthylammonium, le chlorure de triméthylcétylammonium ;
b) le chlorure de stéaramidopropyldiméthyl(myristyl acétate)ammonium de formule : 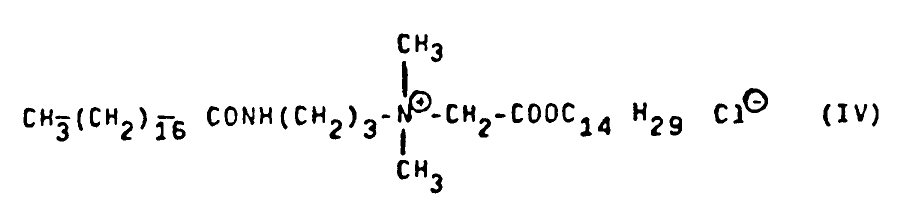 tel que le produit vendu sous la dénomination commerciale "CERAPHYL 70" par la Société "VAN DYK" ;
c) un sel d'ammonium quaternaire de formule : 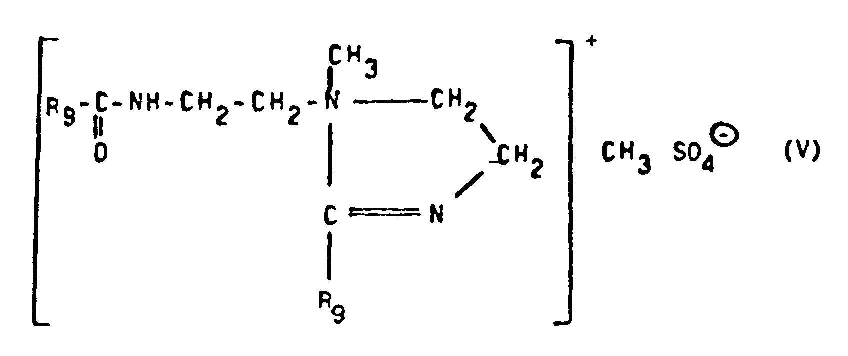 dans lequel R₉ désigne un mélange de radicaux alkényle et/ou alkyle en C₁₃ - C₂₁ dérivé des acides gras du suif, tel que le produit vendu sous la dénomination commerciale "REWOQUAT W 7500" par la Société "REWO".

La protéine quaternisée contenue selon l'invention dans la phase aqueuse continue est avantageusement choisie dans le groupe formé par :
a) certains hydrolysats de protéine porteurs, sur la chaîne polypeptidique, de groupements ammonium quaternaires comportant au moins un radical alkyle en C₁ - C₁₈ et notamment un hydrolysat de protéine animale vendu sous la dénomination commerciale "CROTEIN Q" par la Société "CRODA", dont la chaîne polypeptidique a un poids moléculaire moyen de l'ordre de 12000 ;
b) des hydrolysats de protéine animale portant des groupements triméthylbenzylammonium (dénommés "benzyltrimonium hydrolysed animal protein" dans le "CTFA cosmetic Dictionary" (3ème édition, 1982) publié par "The Cosmetic Toiletry and Fragance Association Inc." et ci-après appelé "dictionnaire CTFA") et, par exemple, celui vendu sous la dénomination commerciale "CROTEIN BTA" par la Société "CRODA" ;
c) des hydrolysats de collagène porteurs de groupements triéthylammonium, dénommés "Triéthonium hydrolysed collagen ethosulfate" dans le dictionnaire CTFA et vendus sous la dénomination commerciale de "QUAT-PRO E" par la Société "MAYBROOK" ;
d) des hydrolysats de collagène porteurs de groupements triméthylammonium et triméthylstéarylammonium, dénommés dans le dictionnaire CTFA "Steartrimonium hydrolysed collagen" et vendus sous la dénomination commerciale "QUAT PRO S" par la Société "MAYBROOK" ;
e) une protéine quaternisée résultant de la condensation de la cocamidopropyl-diméthyl-amine sur une protéine animale hydrolysée, dénommée, dans le supplément de la 3e édition (1982) du dictionnaire CTFA : COCAMIDOPROPYLDIMONIUM HYDROXYPROPYLAMINO HYDROLYSED ANIMAL PROTEIN vendue sous la dénomination commerciale "LEXEIN QX 3000" par la Société "INOLEX".

Les compositions selon l'invention peuvent, de préférence, contenir, à la fois, au moins un tensio-actif cationique et au moins une protéine quaternisée. Dans ce cas :
- l'agent tensio-actif cationique est, de préférence, un chlorure de tétraalkylammonium de formule (I) dans lequel R₁ , R₂ et R₃ sont des radicaux alkyle en C₁ - C₄ et R₄ est un radical alkyle en C₂₀ - C₂₂;
- et la protéine quaternisée est, de préférence, un hydrolysat de protéine animale porteur de groupements ammonium quaternaires comportant un radical alkyle en C₁-C₁₈, dont la chaîne polypeptidique a un poids moléculaire moyen de l'ordre de 12 000.

La phase aqueuse continue de dispersion peut éventuellement contenir, en plus de l'agent tensio-actif cationique et/ou de la protéine quaternisée, au moins un additif connu tel que des conservateurs, des stabilisants, des colorants, des parfums, des adoucissants, des humectants choisis, de préférence, parmi les polyols comme la glycérine, et des épaississants, tels que des alcools gras oxyéthylénés ou non.

Dans la composition selon l'invention, le (ou les) agent(s) tensioactif(s) cationique(s) représente(nt), de préférence, de 0,1 à 6 % en poids, par rapport au poids total de la composition ; la (ou les) protéine(s) quaternisée(s) représente(nt), de préférence, de 0,05 à 0,5 % en poids, par rapport au poids total de la composition ; le (ou les) lipide(s) amphiphile(s) ionique(s) qui constitue(nt) la phase lamellaire représente(nt), de préférence, de 1 % à 10 % en poids, et plus particulièrement 3 à 10 % par rapport au poids total de la composition.

Il est bien entendu que, dans le cas où l'on utilise une grande quantité d'agent(s) tensio-actif(s) cationique(s) par rapport au(x) lipide(s) amphiphile(s) ionique(s), le choix du (ou des) agent(s) cationique(s) n'est pas indifférent et doit être effectué de manière qu'il(s) ne détruise(nt) pas les vésicules.

La composition selon l'invention est généralement préparée par mélange de deux constituants (A) et (B). Le constituant (A) contient la phase lamellaire lipidique hydratée, dans une phase aqueuse continue. Le constituant (B) contient, en phase aqueuse, l'agent cationique et/ou la protéine quaternisée. Chacun des constituants peut renfermer, en outre, divers additifs. On préfère que chacun des constituants (A) et (B) représentent 40 à 60 % en poids par rapport au poids total de la composition ; avantageusement, les deux constituants mélangés ont sensiblement le même poids.

Le constituant (A) est préparé de façon classique et plus particulièrement selon le procédé décrit dans le brevet français n° 2 315 991 :
- dans une première étape, on met en contact avec de l'eau les lipides amphiphiles ioniques, éventuellement mélangés à des additifs destinés à modifier la perméabilité ou la charge des feuillets lipidiques de la phase lamellaire lipidique hydratée que l'on veut former ;
- dans une deuxième étape, on ajoute à la phase lamellaire lipidique hydratée ainsi obtenue une phase aqueuse de dispersion ;
- dans une troisième étape, on soumet le mélange à une agitation énergique pour obtenir des vésicules.

Une fois le constituant (B) préparé, on l'ajoute au constituant (A), sous agitation, jusqu'à complète homogénéisation.

Les compositions selon l'invention sont, de préférence, appliquées sous forme de produits à rincer, avant et plus particulièrement après un shampooing, avant et plus particulièrement après une coloration ou décoloration, avant et plus particulièrement après permanente ou défrisage. Elles peuvent également être appliquées sous forme de produits non rincés, par exemple, avant la mise en plis ou le brushing.

Pour la mise en oeuvre des compositions selon l'invention en vue d'un traitement des cheveux et/ou du cuir chevelu, on applique sur le substrat à traiter une quantité efficace de la composition selon l'invention, on laisse en contact pendant 1 à 15 mn avant de rincer quand il s'agit de produit à rincer. Les quantités de composition appropriées sont, en général, de l'ordre de 20 à 40 g pour une tête dans le cas des produits à rincer et de l'ordre de 5 à 10 g dans le cas des produits non rincés.

Les exemples donnés ci-dessous, permettront de mieux comprendre l'invention.

### Exemple 1:

Dans une première étape , on prépare un constituant (A) comprenant les vésicules. On mélange, en agitant doucement au bain marie à 60°C, 4 g de lécithine de soja à 75% de phosphatidylcholine vendu par la société SEPPIC sous la dénomination "LIPOID S 75", et ceci jusqu'à homogénéisation parfaite (5 mn).

On introduit dans le mélange 8 g d'eau portée à 80°C contenant des conservateurs et l'on mélange pendant environ 5 mn ,on laisse gonfler le mélange pendant 1 heure. A la phase ainsi obtenue, on ajoute 12 g d'eau à 20°C; on agite le mélange quelques minutes puis on affine le mélange par un passage dans un homogénéiseur haute pression à 500 bars (Rannie). On complète par 0,65 g d'eau à 20°C sous agitation.

Dans une seconde étape , on prépare un constituant (B) par un mélange des formulations B1 et B2 suivantes :

| Formulation B1 : | |
|---|---|
| Alcool cétylique / alcool stéarylique (30/70) | 3,36 g |
| Alcool cétylique / alcool stéarylique oxyéthyléné à 33 moles d'oxyde d'éthylène | 0,84 g |
| 2-octyl dodécanol | 0,6 g |
| Alcool cétylique / alcool stéarylique (50/50) | 1,8 g |

| Formulation B2 : | |
|---|---|
| Glycérine | 0,6 g |
| Chlorure de distéaryl diméthyl ammonium | 4,7 g |
| Hydrolysat de collagène à groupements triéthylammonium vendu sous la dénomination "QUAT PRO E" par la société MAYBROOK à 28% de matières actives (=MA) | 0,15 gMA |
| Conservateurs | qs |
| Eau qsp | 68,4 g |

Le mélange des formulations B1 et B2 est effectué sous agitation et on maintient le mélange sous agitation jusqu'à complet refroidissement.

Dans une troisième étape on réalise le mélange du constituant A et du constituant B et on le maintient sous agitation jusqu'à complète homogénéisation.

On applique cette composition à raison d'environ 25 g sur le cuir chevelu et les cheveux lavés et essorés . Après 10 minutes de temps de pose , on rince à l'eau On constate une sensation de fraîcheur à l'application sur le cuir chevelu . Après séchage on voit que les cheveux sont gainés et que la coiffure est légère et gonflante. Les cheveux se démêlent aisément sont brillants doux et lisses jusqu'à leurs pointes.

### Exemple 2:

Dans une première étape , on prépare un constituant (A) comprenant les vésicules. On mélange , en agitant doucement à une température de 60°C , un mélange de 2 g de phospholipides de soja vendu par la société NATTERMANN sous la dénomination "PHOSPHOLIPON 80" et de conservateurs , ceci jusqu'à homogénéisation parfaite (5 mn).

On introduit dans le mélange 4 g d'eau portée à 80°C contenant un conservateur et l'on mélange pendant environ 5 mn puis on laisse gonfler le mélange pendant 1 heure. A la phase ainsi obtenue, on ajoute 6 g d'eau à 20°C; on agite le mélange quelques minutes puis on affine le mélange par un passage dans un homogénéiseur haute pression à 500 bars (Rannie) ,on complète par 0,325 g d'eau à 20°C sous agitation.

Dans une seconde étape , on prépare un constituant (B) par un mélange des formulations B1 et B2 suivantes :

| Formulation B1 : | |
|---|---|
| Alcool cétylique / alcool stéarylique (30/70) | 3,92 g |
| Alcool cétylique / alcool stéarylique oxyéthyléné à 33 moles d'oxyde d'éthylène | 0,98 g |
| 2-octyl dodécanol | 0,7 g |
| Alcool cétylique / alcool stéarylique (50/50) | 2,1 g |

| Formulation B2 : | |
|---|---|
| Glycérine | 0,7 g |
| Sel d'ammonium quaternaire vendu à 75% de MA par la société REWO sous la dénomination commerciale "REWOQUAT 7500 PG" | 5 g MA |
| Proténe quaternisée résultant de la condensation de la cocamidopropyl-diméthyl-amine sur une protéine animale hydrolyée, vendu sous la dénomination commerciale "LEXEIN QX 3000" à 30% de MA par la société INOLEX | 0,2 g MA |
| Conservateurs | qs |
| Eau qsp | 79,8 g |

Le mélange des formulations B1 et B2 est effectué sous agitation et on maintient le mélange sous agitation jusqu'à complet refroidissement.

Dans une troisième étape , on réalise le mélange du constituant A et du constituant B et on le maintient sous agitation jusqu'à complète homogénéisation.

On applique cette composition à raison d'environ 25 g sur le cuir chevelu et les cheveux lavés et essorés . Après 10 minutes de temps de pose , on rince à l'eau . On constate une sensation de fraîcheur à l'application sur le cuir chevelu . Après séchage , on voit que les cheveux sont gainés et que la coiffure est légère et gonflante. Les cheveux se démêlent aisément , sont brillants , doux et lisses jusqu'à leurs pointes.

### Exemple 3:

Dans une première étape , on prépare un constituant (A) comprenant les vésicules. On fond , en agitant doucement à une température de 85°C 4 g de phytostérol polyoxyéthyléné à 5 moles d'oxyde d'éthylène vendu par la société NIKKO sous la dénomination"GENEROL 122 E 5 ". Puis au mélange fondu , on additionne , un mélange de 6 g de lécithine hydrogénée à 30-35% de phosphatidylcholine hydrogénée vendu par la société NIKKO sous la dénomimation "LECINOL S 10", et ceci jusqu'à homogénéisation parfaite (5 mn).

On introduit dans le mélange fondu 20 g d'eau portée à 80°C contenant un conservateur et l'on mélange pendant environ 5 mn,puis on laisse gonfler le mélange pendant 1 heure . A la phase ainsi obtenue, on ajoute 30 g d'eau à 20°C; on agite le mélange quelques minutes , on affine le mélange par un passage dans un homogénéiseur haute pression à 500 bars (Rannie ou Gaulin) puis on complète par 9,3 g d'eau à 20°C sous agitation.

Dans une seconde étape , on prépare un constituant (B) par un mélange des formulations B1 et B2 suivantes:

| Formulation B1 : | |
|---|---|
| Alcool cétylique / alcool stéarylique (30/70) | 2,24 g |
| Alcool cétylique / alcool stéarylique oxyéthyléné à 33 moles d'oxyde d'éthylène | 0,56 g |
| 2-octyl dodécanol | 0,4 g |
| Alcool cétylique / alcool stéarylique (50/50) | 1,2 g |

| Formulation B2 : | |
|---|---|
| Glycérine | 0,4 g |
| Chlorure de béhényl triméthylammonium à 80% de MA vendu par la société HOECHST sous la dénomination "GENAMIN KDM F" | 2,75 g MA |
| Hydrolysat de protéine , comportant une chaîne polypeptidique ayant un poids moléculaire d'environ 12.000 et des groupements ammonium quaternaires porteurs d'au moins un groupe alkyle en C₁-C₁₈, vendu sous la dénomination commerciale "CROTEIN Q" par la société CRODA | 0,125 g |
| Conservateurs | qs |
| Eau qsp | 25,6 g |

Le mélange des formulations B1 et B2 est effectué sous agitation et on maintient le mélange sous agitation jusqu'à complet refroidissement.

Dans une troisième étape , on réalise le mélange du constituant A et du constituant B et on le maintient sous agitation jusqu'à complète homogénéisation.

On applique cette composition à raison d'environ 25 g sur le cuir chevelu et les cheveux lavés et essorés . Après 10 minutes de temps de pose, on rince à l'eau . On constate une sensation de fraîcheur à l'application sur le cuir chevelu . Après séchage , on voit que les cheveux sont gainés et que la coiffure est légère et gonflante. Les cheveux se démêlent aisément , sont brillants , doux et lisses jusqu'à leurs pointes.

### Exemple 4:

Dans une première étape , on prépare un constituant (A) comprenant les vésicules. On mélange , en agitant doucement au bain marie à 60°C, 6 g de lécithine de soja à 75% de phosphatidylcholine vendu par la société SEPPIC sous la dénomination LIPOID S 75 et ceci jusqu'à homogénéisation parfaite (5 mn).

On introduit dans le mélange 12 g d'eau portée à 80°C contenant des conservateurs et l'on mélange pendant environ 5 mm ; on laisse gonfler le mélange pendant 1 heure. A la phase ainsi obtenue, on ajoute 18 g d'eau à 20°C; on agite le mélange quelques minutes puis on affine le mélange par un passage dans un homogénéiseur haute pression à 500 bars (Rannie).On complète à 50 g d'eau à 20 °C sous agitation .

Dans une seconde étape , on prépare un constituant (B) par un mélange des formulations B1 et B2 suivantes:

| Formulation B1 : | |
|---|---|
| Alcool cétylique / alcool stéarylique (30/70) | 3,36 g |
| Alcool cetylique / alcool stéarylique oxyéthyléné à 33 moles d'oxyde d'éthylène | 0,84 g |
| 2-octyl dodécanol | 0,6 g |
| Alcool cétylique / alcool stéarylique (50/50) | 1,8 g |

| Formulation B2 : | |
|---|---|
| Glycérine | 0,6 g |
| Hydrolysat de collagène à groupements triméthylammonium et triméthylstéarylammonium vendu sous la dénomination "QUAT PRO S" par la société MAYBROOK à 90% de MA | 0,5 gMA |
| Conservateurs | qs |
| Eau qsp | 50 g |

Le mélange des formulations B1 et B2 est effectué sous agitation et on maintient le mélange sous agitation jusqu'à complet refroidissement.

Dans une troisième étape , on réalise le mélange du constituant A et du constituant B et on le maintient sous agitation jusqu'à complète homogénéisation .

Cette composition est appliquée sur le cuir chevelu et les cheveux lavés et essorés ; elle a les même avantage que celle obtenue à l'exemple 1.

### Exemple 5:

On prépare une crème antipelliculaire:

Dans une première étape , on prépare un constituant (A) comprenant les vésicules. On mélange , en agitant doucement à une température de 60°C , 4 g de phospholipides de soja vendu par la société NATTERMANN sous la dénomination PHOSPHOLIPON 80 , 0,2 g du sel d'éthanolamine de l'hydroxy-1 méthyl-4 (triméthyl-2,4,4)-pentyl-6 1H-pyridone-2 vendu sous la dénomination OCTOPIROX par la société HOECHST et des conservateurs , ceci jusqu'à homogénéisation parfaite (5 mn).

On introduit dans le mélange 8 g d'eau portée à 80°C contenant un conservateur et l'on mélange pendant environ 5 mn puis on laisse gonfler le mélange pondant 1 heure. A la phase ainsi obtenue, on ajoute 12 g d'eau à 20°C; on agite le mélange quelques minutes puis on affine le mélange par un passage dans un homogénéiseur haute pression à 500 bars (Rannie) ,on complète à 25 g d'eau à 20 °C sous agitation .

Dans une seconde étape , on prépare un constituant (B) par un mélange des formulations B1 et B2 suivantes :

| Formulation B1 : | |
|---|---|
| Alcool cétylique / alcool stéarylique (30/70) | 3,36 g |
| Alcool cétylique / alcool stéarylique oxyéthyléné à 33 moles d'oxyde d'éthylène | 0,84 g |
| 2-octyl dodécanol | 0,6 g |
| Alcool cétylique / alcool stéarylique (50/50) | 1,8 g |

| Formulation B2 : | |
|---|---|
| Glycérine | 0,6 g |
| Hydrolysat de collagène à groupements triéthylammonium vendu sous la dénomination "QUAT PRO E" par la société MAYBROOK à 28% de MA | 0,25 gMA |
| Sel d'ammonium quaternaire vendu à 75% de MA par la société REWO sous la dénomination commerciale "REWOQUAT 7500 PG" | 2 g MA |
| Conservateurs | qs |
| Eau qsp | 75 g |

Le mélange des formulations B1 et B2 est effectué sous agitation et on maintient le mélange sous agitation jusqu'à complet refroidissement.

Dans une troisième étape , on réalise le mélange du constituant A et du constituant B et on le maintient sous agitation jusqu'à complète homogénéisation.

Après des applications bihebdomadaires pendant 3 mois , on observe une régression du nombre des pellicules.

L'effet cosmétique sur le cheveu est le même que celui décrit dans l'exemple 1.

### Exemple 6:

Dans une première étape , on prépare un constituant (A) comprenant les vésicules. On fond , en agitant doucement à une température de 85°C 3,04 g de phytostérol polyoxyéthyléné à 5 moles d'oxyde d'éthylène vendu par la société NIKKO sous la dénomination GENEROL 122 E 5. Puis au mélange fondu , on additionne , 4,56 g de lécithine hydrogénée à 30-35% de phosphatidylcholine hydrogénée vendu par la société NIKKO sous la dénomination LECINOL S 10 , et ceci jusqu'à homogénéisation parfaite (5 mn).

On introduit dans le mélange fondu 15,2 g d'eau portée à 80°C contenant un conservateur et l'on mélange pendant environ 5 mn,puis on laisse gonfler le mélange pondant 1 heure. A la phase ainsi obtenue, on ajoute 22,8 g d'eau à 20°C; on agite le mélange quelques minutes , on affine le mélange par un passage dans un homogénéiseur haute pression à 500 bars (Rannie ou Gaulin) puis on complète à 50 g d'eau à 20 °C sous agitation .

Dans une seconde étape , on prépare un constituant (B) par un mélange des formulations B1 et B2 suivantes :

| Formulation B1 : | |
|---|---|
| Alcool cétylique / alcool stéarylique (30/70) | 3,36 g |
| Alcool cétylique / alcool stéarylique oxyéthyléné à 33 moles d'oxyde d'éthylène | 0,84 g |
| 2-octyl dodécanol | 0,6 g |
| Alcool cétylique / alcool stéarylique (50/50) | 1,8 g |

| Formulation B2 : | |
|---|---|
| Glycérine | 0,6 g |
| Chlorure de triméthylcétylammonium à 25% de MA | 1 gMA |
| Conservateurs | qs |
| Eau qsp | 50 g |

Le mélange des formulations B1 et B2 est effectué sous agitation et on maintient le mélange sous agitation jusqu'à complet refroidissement.

Dans une troisième étape , on réalise le mélange du constituant A et du constituant B et on le maintient sous agitation jusqu'à complète homogénéisation.

Cette composition est appliquée sur le cuir chevelu et les cheveux lavés et essorés ; elle a les mêmes avantages que la composition de l'exemple 1.

## Revendications (Revendications pour l'(les) Etat(s) contractant(s) suivant(s): AT, BE, CH, DE, DK, FR, GB, IT, LI, NL, SE)

1. Composition cosmétique ou pharmaceutique pour le traitement des cheveux et du cuir chevelu renfermant des vésicules constituées d'une phase lamellaire hydratée de lipides amphiphiles ioniques, insolubles dans l'eau, éventuellement associés à un stabilisant, lesdites vésicules étant sous forme dispersée dans une phase aqueuse continue contenant :
1) au moins un agent tensio-actif cationique de formule 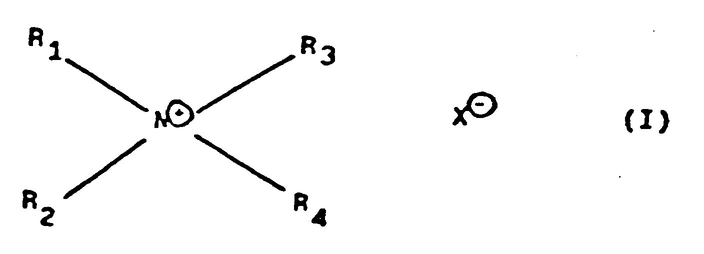 dans laquelle X est le chlore ou CH₃SO⁻₄ et R₁ est un radical alkyle en C₁ - C₄ et dans laquelle :
a) lorsque X est le chlore :
- ou bien R₂ et R₃ sont des radicaux alkyle en C₁ - C₄ , identiques ou différents de R₁ et entre eux, et R₄ est un radical alkyle en C₁₆ - C₂₂ ;
- ou bien R₂ = R₁ et, dans ce cas :
soit R₃ = R₄ = radical alkyle en C₁₈;
soit R₃ = radical (alkyl C₁₇)amidopropyle et R₄ = radical (alkyl C₁₄)acétate et
b) lorsque X est CH₃SO⁻₄:
- R₂ désigne un radical (alkyle et/ou alkényl)amidoéthyle, dans lequel le radical alkyle et/ou alkényle est un radical en C₁₃- C₂₁ et dérive des acides gras du suif ;
- R₃ et R₄ forment ensemble avec l'azote un cycle 4,5-dihydroimidazole substitué en position 2 ;
et/ou
2) au moins une protéine quaternisée constituée par un polypeptide modifié chimiquement portant en bout de chaîne ou greffé sur celle-ci, au moins un groupement ammonium quaternaire comportant au moins une chaîne alkyle en C₁-C₁₈, le polypeptide étant choisi parmi les hydrolisats de protéine animale ;
sous réserve que, si la composition contient comme agent tensio-actif cationique le chlorure de distéaryldiméthylammonium, ladite composition contienne obligatoirement au moins une protéine quaternisée telle que définie sous 2) ci-dessus.
ledit (lesdits) agent(s) tensioactif(s) cationique(s), lorsqu'il(s) est (sont) présent(s), représentant en poids de 0,05 à 10 % du poids total de la composition, ladite (lesdites) protéine(s) quaternisée(s). lorsqu'elle(s) est (sont) présente(s), représentant en poids de 0,05 à 3 % du poids total de la composition et le(s) lipide(s) amphiphile(s) qui constitue(nt) la phase lamellaire représentant de 0,1 à 20 % en poids du poids total de la composition.

2. Composition selon la revendication 1, caractérisée par le fait que les vésicules ont un diamètre moyen compris entre 0,01 et 5 µm, de préférence entre 0,05 et 0,35 µm.

3. Composition selon l'une des revendications 1 ou 2, caractérisée par le fait que le(s) lipide(s) amphiphile(s) ionique(s) est (sont) choisis(s) dans le groupe formé par les phospholipides naturels ou synthétiques.

4. Composition selon l'une des revendications 1 ou 2, caractérisée par le fait que le(s) lipide(s) amphiphile(s) ionique(s) est (sont) choisi(s) dans le groupe formé par les composés amphotères comportant deux chaînes lipophiles ou une association de deux ions organiques à longue chaîne de signes opposés.

5. Composition selon l'une des revendications 1 ou 2, caractérisée par le fait que le(s) lipide(s) amphiphile(s) ionique(s) est (sont) choisi(s) dans le groupe formé par les composés anioniques.

6. Composition selon la revendication 5, caractérisée par le fait que le(s) lipides amphiphile(s) ionique(s) est (sont) choisi(s) dans le groupe formé par les composés de formule : 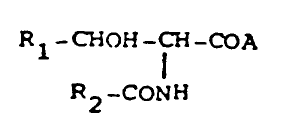 formule dans laquelle :
- R₁ désigne un radical alcoyle ou alcényle en C₇-C₂₁ ;
- R₂ désigne un radical hydrocarboné,saturé ou insaturé,en C₇-C₃₁ ;
- COA désigne un groupement choisi parmi les groupements suivants :
- COOM, M étant H, Na, K, NH₄ ou un ion ammonium substitué dérivé d'une amine ;
- un reste 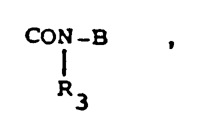 B étant un radical dérivé d'amines primaires ou secondaires, mono- ou polyhydroxylées et R₃ désignant un atome d'hydrogéne ou un radical méthyle, éthyle ou hydroxyéthyle ;
- un reste 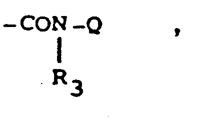 Q désignant un radical amino-alcoyle ou ammonioalcoyle substitué et R₃ ayant la signification indiquée ci-dessus ; et
- COOZ, Z représentant le reste d'un polyol en C₃-C₇.

7. Composition selon l'une des revendications 1 à 6, caractérisée par le fait que le(s) lipide(s) amphiphile(s) ionique(s), qui constitue(nt) la phase lamellaire lipidique hydratée, est (ou sont) associé(s) à au moins un stabilsant choisi dans le groupe formé par les stérols, les sels mono ou disodiques de glutamates d'acyle, le radical acyle étant en C₁₄-C₂₂, les esters phosphoriques d'alcools gras en C₁₂-C₁₆ et les phytostérols oxyéthylénés.

8. Composition selon I'une des revendications 1 à 7, caractérisée par le fait que le(s) lipide(s) amphiphile(s) ionique(s), qui constitue(nt) la phase lamellaire lipidique hydratée, est (sont) associé(s) à au moins un stabilisant anionique en une quantité au plus égale à 12 % en poids et/ou à au moins un stérol en une quantité au plus égale à 100 % en poids, la proportion étant calculée, dans les deux cas, par rapport au poids dudit (ou desdits) lipide(s) amphiphile(s) ionique(s).

9. Composition selon l'une des revendications 1 à 8, caractérisée par le fait que la phase lamellaire lipidique hydratée contient de l'eau en présence ou non d'un agent cosmétiquement ou pharmaceutiquement actif.

10. Composition selon la revendication 9, dans laquelle la phase lamellaire lipidique hydratée contient un agent actif, caractérisée par le fait que l'agent actif est choisi dans le groupe formé par les agents anti-chute ou de repousse des cheveux, les rétinoïdes et apparente, les anti-inflammatoires, les anti-fongiques, les anti-séborrhéiques et les filtres solaires.

11. Composition selon l'une des revendications 1 à 10, caractérisée par le fait que l'agent tensio-actif cationique contenu dans ta phase aqueuse continue est choisi dans le groupe forme par :
a) les halogénures de tétraalkylammonium ;
b) le chlorure de stéaramidopropyl-diméthyl (myristyl acétate) ammonium de formule : 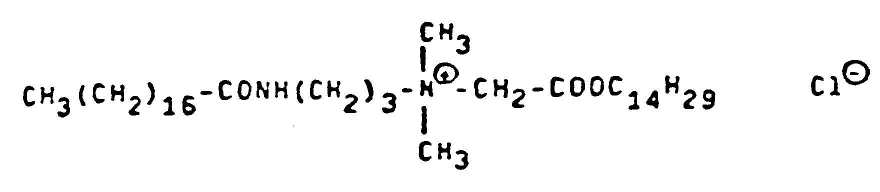
c) un sel d'ammonium quaternaire de formule : 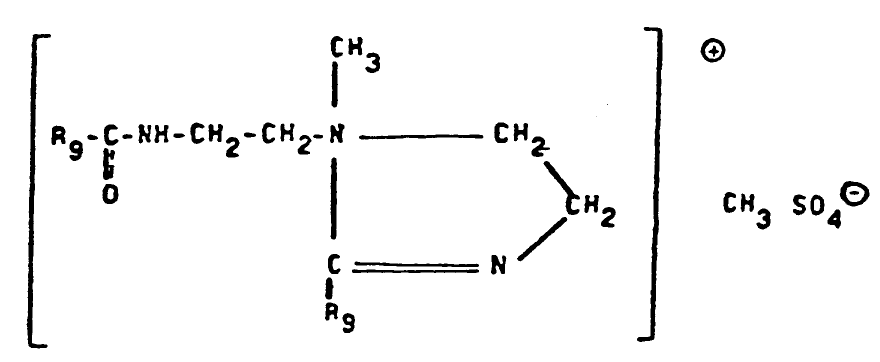 dans lequel R₉ désigne un mélange de radicaux alkényle et/ou alkyle en C₁₃ - C₂₁ dérivé des acides gras du suif.

12. Composition selon l'une des revendications 1 à 11, caractérisée par le fait que la protéine quaternisée est choisie dans le groupe formé par :
a) des hydrolysats de protéine animale portant sur la chaîne polypeptidique des groupements ammonium quaternaires comportant au moins un radical alkyle en C₁ - C₁₈:
b) des hydrolysats de protéine animale portant des groupements triméthylbenzylammonium ;
c) des hydrolysats de collagène porteurs de groupements triéthylammonium ;
d) des hydrolysats de collagène porteurs de groupements triméthylammonium et triméthylstéarylammonium ;
e) une protéine quaternisée résultant de la condensation de la cocamidopropyl-diméthyl-amine sur une protéine animale hydrolysée.

13. Composition selon l'une des revendications 1 à 12, caractérisée par le fait qu'elle contient à la fois au moins un agent tensio-actif cationique et au moins une protéine quaternisée.

14. Composition selon la revendication 13, caractérisée par le fait qu'elle contient d'une part, du chlorure de béhényltriméthylammonium et d'autre part, un hydrolysat de protéine animale porteur sur la chaîne polypeptidique de groupements ammonium quaternaires comportant au moins une chaîne alkyle en C₁ - C₁₈, la chaîne polypeptidique ayant un poids moléculaire moyen d'environ 12.000.

15. Composition selon l'une des revendications 1 à 14, caractérisée par le fait que la phase aqueuse continue contient au moins un additif pris dans le groupe formé par les conservateurs, les stabilisants, les colorants, les humectants, les adoucissants, les parfums et les épaississants.

16. Composition selon l'une des revendications 1 à 15, contenant au moins un agent tensio-actif cationique, caractérisée par le fait que ledit agent tensicactif cationique représente en poids de 0,1 à 6 % du poids total de la composition.

17. Composition selon l'une des revendications 1 à 16, contenant au moins une protéine quaternisée, caractérisée par le fait que ladite protéine quaternisée représente en poids de 0,05 à 0,5 % du poids total de la composition.

18. Composition selon l'une des revendications 1 à 17, caractérisée par le fait que le(s) lipide(s) amphiphile(s) ionique(s), qui constitue(nt) la phase lamellaire, représente(nt) 1 % à 10 % en poids et, plus pariculièrement, de 3 % à 10 % en poids, du poids total de la composition.

19. Procédé de traitement des cheveux et du cuir chevelu, caractérisé par le fait qu'on applique sur la tête 20 à 40 g d'une composition cosmétique selon l'une des revendications 1 à 18, qu'on laisse en contact pendant 1 à 15 mn, puis qu'on rince à l'eau.

20. Procédé de traitement des cheveux et du cuir chevelu, caractérisé par le fait qu'on applique sur la tête 5 à 10 g d'une composition cosmétique selon l'une des revendications 1 à 18.

## Revendications (Revendications pour l'(les) Etat(s) contractant(s) suivant(s): ES, GR)

1. Composition cosmétique pour le traitement des cheveux et du cuir chevelu renfermant des vésicules constituées d'une phase lamellaire hydratée de lipides amphiphiles ioniques, insolubles dans l'eau, éventuellement :associés à un stabilisant, lesdites vésicules étant sous forme dispersée dans une phase aqueuse continue contenant :
1) au moins un agent tensio-actif cationique de formule 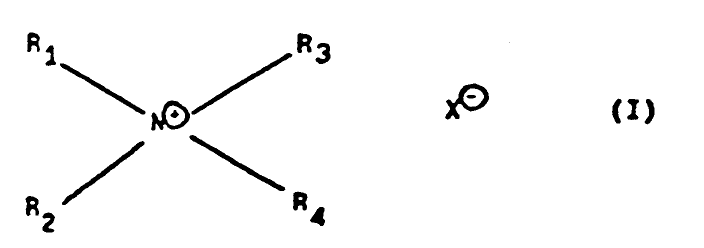 dans laquelle X est le chlore ou CH₃SO⁻₄ et R₁ est un radical alkyle en C₁ - C₄ et dans laquelle :
a) lorsque X est le chlore :
- ou bien R₂ et R₃ sont des radicaux alkyle en C₁ - C₄ , identiques ou différents de R₁ et entre eux, et R₄ est un radical alkyle en C₁₆ - C₂₂ ;
- ou bien R₂ = R₁ et, dans ce cas :
soit R₃ = R₄ = radical alkyle en C₁₈;
soit R₃ = radical (alkyl C₁₇)amidopropyle et R₄ = radical (alkyl C₁₄)acétate et
b) lorsque X est CH₃SO⁻₄ :
- R₂ désigne un radical (alkyle et/ou alkényl)amidoéthyle, dans lequel le radical alkyle et/ou alkényle est un radical en C₁₃- C₂₁ et dérive des acides gras du suif ;
- R₃ et R₄ forment ensemble avec l'azote un cycle 4,5-dihydroimidazole substitué en position 2 ;
et/ou
2) au moins une protéine quaternisée constituée par un polypeptide modifié chimiquement portant en bout de chaîne ou greffé sur celle-ci, au moins un groupement ammonium quaternaire comportant au moins une chaîne alkyle en C₁-C₁₈, le polypeptide étant choisi parmi les hydrolisats de protéine animale ;
sous réserve que, si la composition contient comme agent tensio-actif cationique le chlorure de distéaryldiméthylammonium, ladite composition contienne obligatoirement au moins une protéine quaternisée telle que définie sous 2) ci-dessus.
ledit (lesdits) agent(s) tensioactif(s) cationique(s), lorsqu'il(s) est (sont) présent(s), représentant en poids de 0,05 à 10 % du poids total de la composition, ladite (lesdites) protéine(s) quaternisée(s). lorsqu'elle(s) est (sont) présente(s), représentant en poids de 0,05 à 3 % du poids total de la composition et le(s) lipide(s) amphiphile(s) qui constitue(nt) la phase lamellaire représentant de 0,1 à 20 % en poids du poids total de la composition.

2. Composition selon la revendication 1, caractérisée par le fait que les vésicules ont un diamètre moyen compris entre 0,01 et 5 µm, de préférence entre 0,05 et 0,35 µm.

3. Composition selon l'une des revendications 1 ou 2, caractérisée par le fait que le(s) lipide(s) amphiphile(s) ionique(s) est (sont) choisis(s) dans le groupe formé par les phospholipides naturels ou synthétiques.

4. Composition selon l'une des revendications 1 ou 2, caractérisée par le fait que le(s) lipide(s) amphiphile(s) ionique(s) est (sont) choisi(s) dans le groupe formé par les composés amphotères comportant deux chaînes lipophiles ou une association de deux ions organiques à longue chaîne de signes opposés.

5. Composition selon l'une des revendications 1 ou 2, caractérisée par le fait que le(s) lipide(s) amphiphile(s) ionique(s) est (sont) choisi(s) dans le groupe formé par les composés anioniques.

6. Composition selon la revendication 5, caractérisée par le fait que le(s) lipides amphiphile(s) ionique(s) est (sont) choisi(s) dans le groupe formé par les composés de formule : 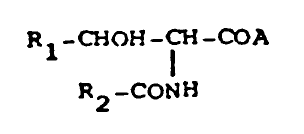 formule dans laquelle :
- R₁ désigne un radical alcoyle ou alcényle en C₇-C₂₁ ;
- R₂ désigne un radical hydrocarboné, saturé ou insaturé, en C₇-C₃₁ ;
- COA désigne un groupement choisi parmi les groupements suivants :
- COOM, M étant H, Na, K, NH₄ ou un ion ammonium substitué dérivé d'une amine ;
- un reste 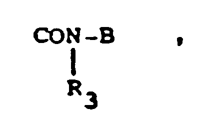 B étant un radical dérivé d'amines primaires ou secondaires, mono- ou polyhydroxylées et R₃ désignant un atome d'hydrogène ou un radical méthyle, éthyle ou hydroxyéthyle ;
- un reste 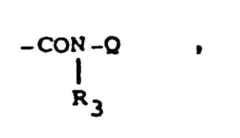 Q désignant un radical amino-alcoyle ou ammonioalcoyle substitué et R₃ ayant la signification indiquée ci-dessus ; et
- COOZ, Z représentant le reste d'un polyol en C₃-C₇.

7. Composition selon l'une des revendications 1 à 6, caractérisée par le fait que le(s) lipide(s) amphiphile(s) ionique(s), qui constitue(nt) la phase lamellaire lipidique hydratée, est (ou sont) associé(s) à au moins un stabilisant choisi dans le groupe formé par les stérols, les sels mono ou disodiques de glutamates d'acyle, le radical acyle étant en C₁₄-C₂₂, les esters phosphoriques d'alcools gras en C₁₂-C₁₆ et les phytostérols oxyéthylénés.

8. Composition selon l'une des revendications 1 à 7, caractérisée par le fait que le(s) lipide(s) amphiphile(s) ionique(s), qui constitue(nt) la phase lamellaire lipidique hydratée, est (sont) associé(s) à au moins un stabilisant anionique en une quantité au plus égale à 12 % en poids et/ou à au moins un stérol en une quantité au plus égale à 100 % en poids, la proportion étant calculée, dans les deux cas, par rapport au poids dudit (ou desdits) lipide(s) amphiphile(s) ionique(s).

9. Composition selon l'une des revendications 1 à 8, caractérisée par le fait que la phase lamellaire lipidique hydratée contient de l'eau en présence ou non d'un agent cosmétiquement actif.

10. Composition selon la revendication 9, dans laquelle la phase lamellaire lipidique hydratée contient un agent actif, caractérisée par le fait que l'agent actif est choisi dans le groupe formé par les agents anti-chute ou de repousse des cheveux, les rétinoîdes et apparentés, les anti-inflammatoires, les anti-fonqiques, les anti-séborrhéiques et les filtres solaires.

11. Composition selon l'une des revendications 1 à 10, caractérisée par le fait que l'agent tensio-actif cationique contenu dans la phase aqueuse continue est choisi dans le groupe formé par :
a) les halogénures de tétraalkylammonium ;
b) le chlorure de stéaramidopropyl-diméthyl (myristyl acétate) ammonium de formule : 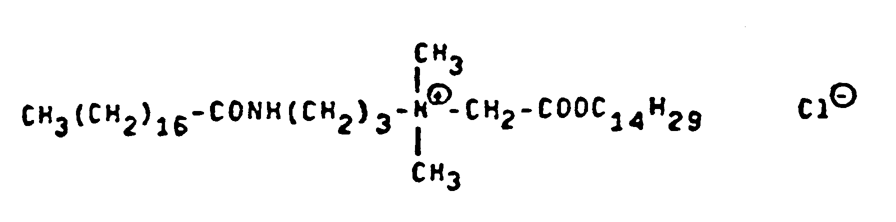
c) un sel d'ammonium quaternaire de formule : 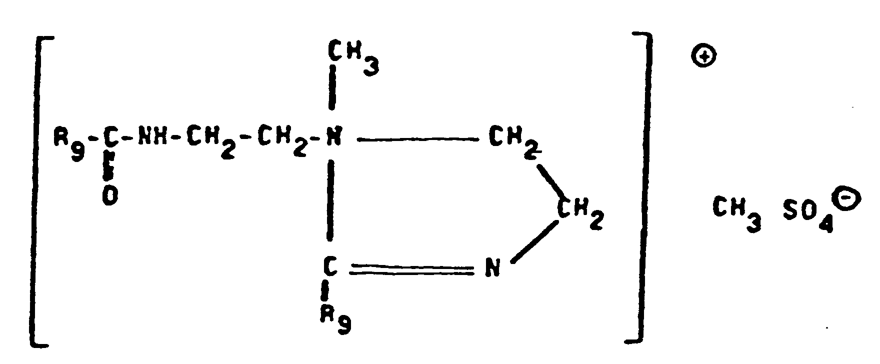 dans lequel R₉ dédigne un mélange de radicaux alkényle et/ou alkyle en C₁₃ - C₂₁ dérivé des acides gras du suif.

12. Composition selon l'une des revendications 1 à 11, caractérisée par le fait que la protéine quaternisée est choisie dans le groupe formé par :
a) des hydrolysats de protéine animale portant sur la chaîne polypeptidique des groupements ammonium quaternaires comportant au moins un radical alkyle en C₁ - C₁₈;
b) des hydrolysats de protéine animale portant des groupements triméthylbenzylammonium ;
c) des hydrolysats de collagène porteurs de groupements triéthylammonium ;
d) des hydrolysats de collagène porteurs de groupements triméthylammonium et triméthylstéarylammonium ;
e) une protéine quaternisée résultant de la condensation de la cocamidopropyl-diméthyl-amine sur une protéine animale hydrolysée.

13. Composition selon l'une des revendications 1 à 12, caractérisée par le fait qu'elle contient à la fois au moins un agent tensio-actif cationique et au moins une protéine quaternisée.

14. Composition selon la revendication 13, caractérisée par le fait qu'elle contient d'une part, du chlorure de béhényltriméthylammonium et d'autre part, un hydrolysat de protéine animale porteur sur la chaîne polypeptidique de groupements ammonium quaternaires comportant au moins une chaîne alkyle en C₁ - C₁₈, la chaîne polypeptidique ayant un poids moléculaire moyen d'environ 12.000.

15. Composition selon l'une des revendications 1 à 14, caractérisée par le fait que la phase aqueuse continue contient au moins un additif pris dans le groupe formé par les conservateurs, les stabilisants, les colorants, les humectants, les adoucissants, les parfums et les épaississants.

16. Composition selon l'une des revendications 1 à 15, contenant au moins un agent tensio-actif cationique, caractérisée par le fait que ledit agent tensioactif cationique représente en poids de 0,1 à 6 % du poids total de la composition.

17. Composition selon l'une des revendications 1 à 16, contenant au moins une protéine quaternisée, caractérisée par le fait que ladite protéine quaternisée représente en poids de 0,05 à 0,5 % du poids total de la composition.

18. Composition selon l'une des revendications 1 à 17, caractérisée par le fait que le(s) lipide(s) amphiphile(s) ionique(s), qui constitue(nt) la phase lamellaire, représente(nt) 1 % à 10 % en poids et, plus particulièrement, de 3 % à 10 % en poids, du poids total de la composition.

19. Procédé de traitement des cheveux et du cuir chevelu, caractérisé par le fait qu'on applique sur la tête 20 à 40 g d'une composition cosmétique selon l'une des revendications 1 à 18, qu'on laisse en contact pendant 1 à 15 mn, puis qu'on rince à l'eau.

20. Procédé de traitement des cheveux et du cuir chevelu, caractérisé par le fait qu'on applique sur la tête 5 à 10 g d'une composition cosmétique selon l'une des revendications 1 à 18.

## Claims (Claims for the following Contracting State(s): AT, BE, CH, DE, DK, FR, GB, IT, LI, NL, SE)

1. Cosmetic or pharmaceutical composition for the treatment of hair and of the scalp, containing vesicles formed of a water insoluble hydrated lamellar phase containing ionic amphiphilic lipids which are optionally used in combination with a stabilizer, the said vesicles being in a dispersed form in a continuous aqueous phase containing:
1) at least one cationic surface-active agent of formula: 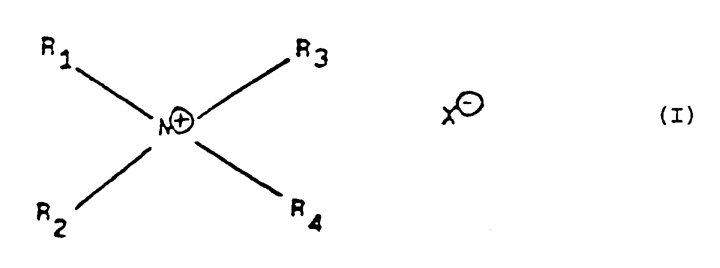 in which X is chlorine or CH₃SO₄⁻ and R₁ is a C₁-C₄ alkyl radical and in which:
a) when X is chlorine,
- either R₂ and R₃ are C₁-C₄ alkyl radicals identical with or different from R₁ and from each other, and R₄ is a C₁₆-C₂₂ alkyl radical;
- or else R₂ = R₁ and, in this case:
either R₃ = R₄ = C₁₈ alkyl radical;
or R₃ = (C₁₇ alkyl)amidopropyl radical and R₄ = (C₁₄ alkyl)acetate radical and
b) when X is CH₃SO₄⁻:
- R₂ denotes an (alkyl and/or alkenyl)amidoethyl radical in which the alkyl and/or alkenyl radical is a C₁₃-C₂₁ radical and is derived from tallow fatty acids;
- R₃ and R₄ together with the nitrogen form a 4,5-dihydroimidazole ring substituted in position 2;
and/or
2) at least one quaternized protein consisting of a chemically modified polypeptide carrying at the end of a chain or grafted onto the latter at least one quaternary ammonium group containing at least one C₁-C₁₈ alkyl chain, the polypeptide being chosen from animal protein hydrolysates;
provided that, if the composition contains distearyldimethylammonium chloride as a cationic surface-active agent, the said composition necessarily contains at least one quaternized protein such as defined under 2) above, the said cationic surface-active agent(s), when present, representing from 0.05 to 10 % by weight of the total weight of the composition, the said quaternized protein(s), when present, representing from 0.05 to 3 % by weight of the total weight of the composition and the amphiphilic lipid(s) which constitutes (constitute) the lamellar phase representing from 0.1 to 20 % by weight of the total weight of the composition.

2. Composition according to Claim 1, characterized in that the vesicles have an average diameter of between 0.01 and 5 µm, preferably between 0.05 and 0.35 µm.

3. Composition according to one of Claims 1 or 2, characterized in that the ionic amphiphilic lipid(s) is(are) chosen from the group made up of natural or synthetic phospholipids.

4. Composition according to one of Claims 1 or 2, characterized in that the ionic amphiphilic lipid(s) is(are) chosen from the group made up of amphoteric compounds containing two lipophilic chains or a combination of two long-chain organic ions of opposite signs.

5. Composition according to one of Claims 1 or 2, characterized in that the ionic amphiphilic lipid(s) is(are) chosen from the group made up of anionic compounds.

6. Composition according to Claim 5, characterized in that the ionic amphiphilic lipid(s) is(are) chosen from the group made up from the compounds of formula: 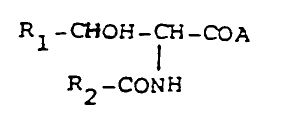 in which formula:
- R₁ denotes a C₇-C₂₁ alkyl or alkenyl radical;
- R₂ denotes a C₇-C₃₁ saturated or unsaturated hydrocarbon radical;
- COA denotes a group chosen from the following groups:
- COOM, M being H, Na, K, NH₄ or a substituted ammonium ion derived from an amine;
- a residue 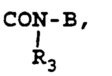 B being a radical derived from mono- or polyhydroxylated primary or secondary amines, and R₃ denoting a hydrogen atom or a methyl, ethyl or hydroxyethyl radical;
- a residue 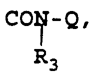 Q denoting a substituted aminoalkyl or ammonioalkyl radical and R₃ having the meaning shown above; and
- COOZ, Z denoting the residue of a C₃-C₇ polyol.

7. Composition according to one of Claims 1 to 6, characterized in that the ionic amphiphilic lipid(s) which constitutes (constitute) the hydrated lipidic lamellar phase is(or are) used in combination with at least one stabilizer chosen from the group made up of sterols, mono- or disodium salts of acyl glutamates, the acyl radical being C₁₄-C₂₂, phosphoric esters of C₁₂-C₁₆ fatty alcohols and oxyethylenated phytosterols.

8. Composition according to one of Claims 1 to 7, characterized in that the ionic amphiphilic lipid(s) which constitutes (constitute) the hydrated lipidic lamellar phase is (are) used in combination with at least one anionic stabilizer in a quantity not exceeding 12 % by weight and/or with at least one sterol in a quantity not exceeding 100 % by weight, the proportion in both cases being calculated relative to the weight of the said ionic amphiphilic lipid(s).

9. Composition according to one of Claims 1 to 8, characterized in that the hydrated lipidic lamellar phase contains water in the presence or absence of a cosmetically or pharmaceutically active agent.

10. Composition according to Claim 9, in which the hydrated lipidic lamellar phase contains an active agent, characterized in that the active agent is chosen from the group made up of hair loss-preventing or growth-stimulating agents, retinoids and related substances, antiinflammatories, antifungals, antiseborrhoeics and sunscreens.

11. Composition according to one of Claims 1 to 10, characterized in that the cationic surface-active agent present in the continuous aqueous phase is chosen from the group made up of:
a) tetraalkylammonium halides;
b) stearamidopropyldimethyl(myristylacetate)-ammonium chloride of formula: 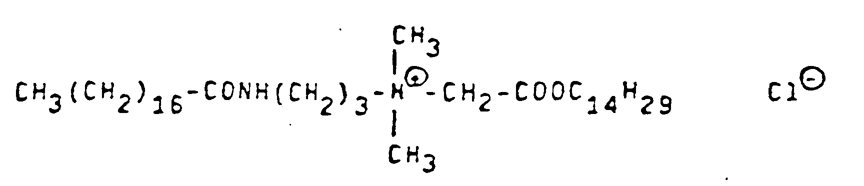
c) a quaternary ammonium salt of formula: 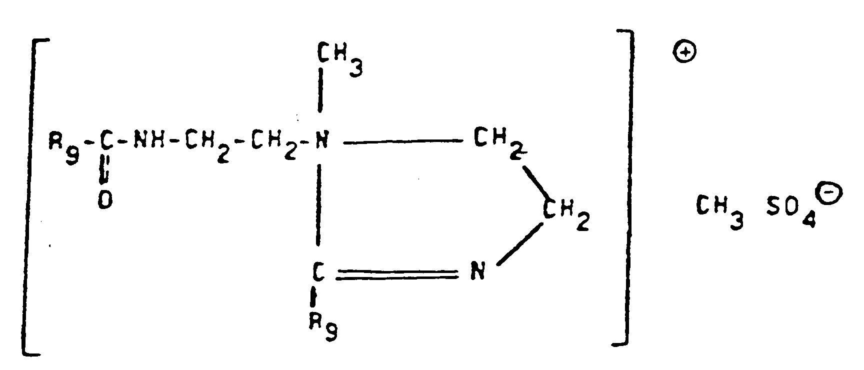 in which R₉ denotes a mixture of C₁₃-C₂₁ alkenyl and/or alkyl radicals derived from tallow fatty acids.

12. Composition according to one of Claims 1 to 11, characterized in that the quaternized protein is chosen from the group made up of:
a) animal protein hydrolysates carrying, on the polypeptide chain, quaternary ammonium groups containing at least one C₁-C₁₈ alkyl radical;
b) animal protein hydrolysates carrying trimethylbenzylammonium groups;
c) collagen hydrolysates carrying triethylammonium groups;
d) collagen hydrolysates carrying trimethylammonium and trimethylstearylammonium groups;
e) a quaternized protein resulting from the condensation of cocamidopropyl-dimethyl-amine onto a hydrolysed animal protein.

13. Composition according to one of Claims 1 to 12, characterized in that it contains at least one cationic surface-active agent and at least one quaternized protein at the same time.

14. Composition according to Claim 13, characterized in that it contains, on the one hand, behenyltrimethylammonium chloride and, on the other hand, an animal protein hydrolysate carrying, on the polypeptide chain, quaternary ammonium groups containing at least one C₁-C₁₈ alkyl chain, the polypeptide chain having an average molecular weight of approximately 12,000.

15. Composition according to one of Claims 1 to 14, characterized in that the continuous aqueous phase contains at least one additive taken from the group made up of preserving agents, stabilizers, colorants, humectants, softeners, perfumes and thickeners.

16. Composition according to one of Claims 1 to 15, containing at least one cationic surface-active agent, characterized in that the said cationic surface-active agent represents from 0.1 to 6 % by weight of the total weight of the composition.

17. Composition according to one of Claims 1 to 16, containing at least one quaternized protein, characterized in that the said quaternized protein represents from 0.05 to 0.5 % by weight of the total weight of the composition.

18. Composition according to one of Claims 1 to 17, characterized in that the ionic amphiphilic lipid(s) which constitutes (constitute) the lamellar phase represents (represent) 1 % to 10 % by weight and, more particularly, from 3 % to 10 % by weight, of the total weight of the composition.

19. Process for treating hair and the scalp, characterized in that 20 to 40 g of a cosmetic composition according to one of Claims 1 to 18 are applied to the head, are left in contact for 1 to 15 min and are then rinsed off with water.

20. Process for the treatment of hair and of the scalp, characterized in that 5 to 10 g of a cosmetic composition according to one of Claims 1 to 18 are applied to the head.

## Claims (Claims for the following Contracting State(s): ES, GR)

1. Cosmetic composition for the treatment of hair and of the scalp, containing vesicles formed of a water insoluble hydrated lamellar phase containing ionic amphiphilic lipids which are optionally used in combination with a stabilizer, the said vesicles being in a dispersed form in a continuous aqueous phase containing:
1) at least one cationic surface-active agent of formula: 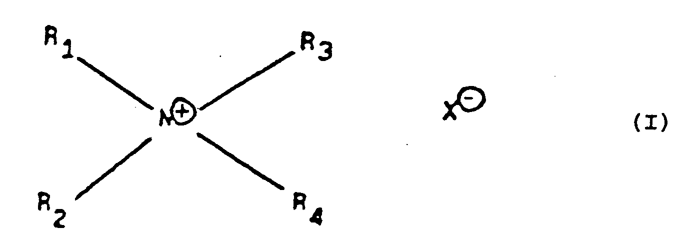 in which X is chlorine or CH₃SO₄⁻ and R₁ is a C₁-C₄ alkyl radical and in which:
a) when X is chlorine,
- either R₂ and R₃ are C₁-C₄ alkyl radicals identical with or different from R₁ and from each other, and R₄ is a C₁₆-C₂₂ alkyl radical;
- or else R₂ = R₁ and, in this case:
either R₃ = R₄ = C₁₈ alkyl radical;
or R₃ = (C₁₇ alkyl)amidopropyl radical and R₄ = (C₁₄ alkyl)acetate radical and
b) when X is CH₃SO₄⁻:
- R₂ denotes an (alkyl and/or alkenyl)amidoethyl radical in which the alkyl and/or alkenyl radical is a C₁₃-C₂₁ radical and is derived from tallow fatty acids;
- R₃ and R₄ together with the nitrogen form a 4,5-dihydroimidazole ring substituted in position 2;
and/or
2) at least one quaternized protein consisting of a chemically modified polypeptide carrying at the end of a chain or grafted onto the latter at least one quaternary ammonium group containing at least one C₁-C₁₈ alkyl chain, the polypeptide being chosen from animal protein hydrolysates;
provided that, if the composition contains distearyldimethylammonium chloride as a cationic surface-active agent, the said composition necessarily contains at least one quaternized protein such as defined under 2) above, the said cationic surface-active agent(s), when present, representing from 0.05 to 10 % by weight of the total weight of the composition, the said quaternized protein(s), when present, representing from 0.05 to 3 % by weight of the total weight of the composition and the amphiphilic lipid(s) which constitutes (constitute) the lamellar phase representing from 0.1 to 20 % by weight of the total weigh£ of the composition.

2. Composition according to Claim 1, characterized in that the vesicles have an average diameter of between 0.01 and 5 µm, preferably between 0.05 and 0.35 µm.

3. Composition according to one of Claims 1 or 2, characterized in that the ionic amphiphilic lipid(s) is(are) chosen from the group made up of natural or synthetic phospholipids.

4. Composition according to one of Claims 1 or 2, characterized in that the ionic amphiphilic lipid(s) is(are) chosen from the group made up of amphoteric compounds containing two lipophilic chains or a combination of two long-chain organic ions of opposite signs.

5. Composition according to one of Claims 1 or 2, characterized in that the ionic amphiphilic lipid(s) is(are) chosen from the group made up of anionic compounds.

6. Composition according to Claim 5, characterized in that the ionic amphiphilic lipid(s) is(are) chosen from the group made up from the compounds of formula: 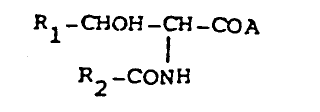 in which formula:
- R₁ denotes a C₇-C₂₁ alkyl or alkenyl radical;
- R₂ denotes a C₇-C₃₁ saturated or unsaturated hydrocarbon radical;
- COA denotes a group chosen from the following groups:
- COOM, M being H, Na, K, NH₄ or a substituted ammonium ion derived from an amine;
- a residue 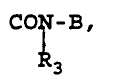 B being a radical derived from mono- or polyhydroxylated primary or secondary amines, and R₃ denoting a hydrogen atom or a methyl, ethyl or hydroxyethyl radical;
- a residue 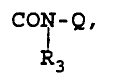 Q denoting a substituted aminoalkyl or ammonioalkyl radical and R₃ having the meaning shown above; and
- COOZ, Z denoting the residue of a C₃-C₇ polyol.

7. Composition according to one of Claims 1 to 6, characterized in that the ionic amphiphilic lipid(s) which constitutes(constitute) the hydrated lipidic lamellar phase is(or are) used in combination with at least one stabilizer chosen from the group made up of sterols, mono- or disodium salts of acyl glutamates, the acyl radical being C₁₄-C₂₂, phosphoric esters of C₁₂-C₁₆ fatty alcohols and oxyethylenated phytosterols.

8. Composition according to one of Claims 1 to 7, characterized in that the ionic amphiphilic lipid(s) which constitutes (constitute) the hydrated lipidic lamellar phase is(are) used in combination with at least one anionic stabilizer in a quantity not exceeding 12 % by weight and/or with at least one sterol in a quantity not exceeding 100 % by weight, the proportion in both cases being calculated relative to the weight of the said ionic amphiphilic lipid(s).

9. Composition according to one of Claims 1 to 8, characterized in that the hydrated lipidic lamellar phase contains water in the presence or absence of a cosmetically active agent.

10. Composition according to Claim 9, in which the hydrated lipidic lamellar phase contains an active agent, characterized in that the active agent is chosen from the group made up of hair loss-preventing or growth-stimulating agents, retinoids and related substances, antiinflammatories, antifungals, antiseborrhoeics and sunscreens.

11. Composition according to one of Claims 1 to 10, characterized in that the cationic surface-active agent present in the continuous aqueous phase is chosen from the group made up of:
a) tetraalkylammonium halides;
b) stearamidopropyldimethyl(myristylacetate)ammonium chloride of formula: 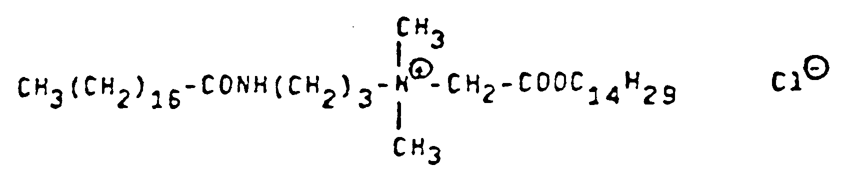
c) a quaternary ammonium salt of formula: 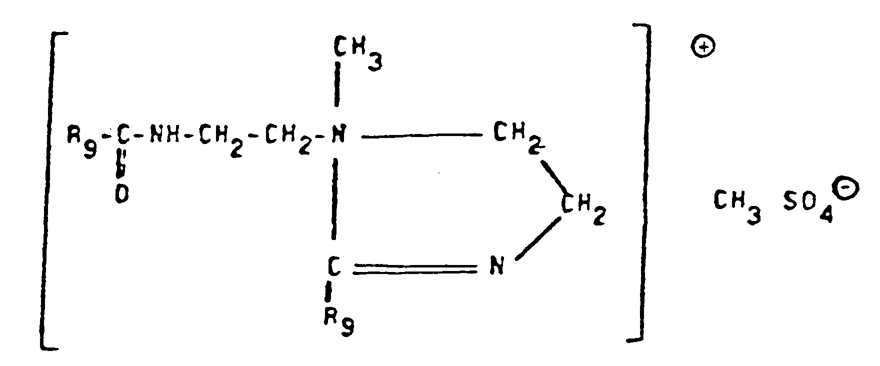 in which R₉ denotes a mixture of C₁₃-C₂₁ alkenyl and/or alkyl radicals derived from tallow fatty acids.

12. Composition according to one of Claims 1 to 11, characterized in that the quaternized protein is chosen from the group made up of:
a) animal protein hydrolysates carrying, on the polypeptide chain, quaternary ammonium groups containing at least one C₁-C₁₈ alkyl radical;
b) animal protein hydrolysates carrying trimethylbenzylammonium groups;
c) collagen hydrolysates carrying triethylammonium groups;
d) collagen hydrolysates carrying trimethylammonium and trimethylstearylammonium groups;
e) a quaternized protein resulting from the condensation of cocamidopropyl-dimethyl-amine onto a hydrolysed animal protein.

13. Composition according to one of Claims 1 to 12, characterized in that it contains at least one cationic surface-active agent and at least one quaternized protein at the same time.

14. Composition according to Claim 13, characterized in that it contains, on the one hand, behenyltrimethylammonium chloride and, on the other hand, an animal protein hydrolysate carrying, on the polypeptide chain, quaternary ammonium groups containing at least one C₁-C₁₈ alkyl chain, the polypeptide chain having an average molecular weight of approximately 12,000.

15. Composition according to one of Claims 1 to 14, characterized in that the continuous aqueous phase contains at least one additive taken from the group made up of preserving agents, stabilizers, colorants, humectants, softeners, perfumes and thickeners.

16. Composition according to one of Claims 1 to 15, containing at least one cationic surface-active agent, characterized in that the said cationic surface-active agent represents from 0.1 to 6 % by weight of the total weight of the composition.

17. Composition according to one of Claims 1 to 16, containing at least one quaternized protein, characterized in that the said quaternized protein represents from 0.05 to 0.5 % by weight of the total weight of the composition.

18. Composition according to one of Claims 1 to 17, characterized in that the ionic amphiphilic lipid(s) which constitutes(constitute) the lamellar phase represents(represent) 1 % to 10 % by weight and, more particularly, from 3 % to 10 % by weight, of the total weight of the composition.

19. Process for treating hair and the scalp, characterized in that 20 to 40 g of a cosmetic composition according to one of Claims 1 to 18 are applied to the head, are left in contact for 1 to 15 min and are then rinsed off with water.

20. Process for the treatment of hair and of the scalp, characterized in that 5 to 10 g of a cosmetic composition according to one of Claims 1 to 18 are applied to the head.

## Patentansprüche (Patentansprüche für folgende(n) Vertragsstaat(en): AT, BE, CH, DE, DK, FR, GB, IT, LI, NL, SE)

1. Kosmetisches oder pharmazeutisches Mittel zur Behandlung der Haare und der Kopfhaut, das Vesikeln enthält welche von einem hydratisierten in Wasser unlöslichen lamellaren Lipidphase gebildet sind, wobei die Lipidphase ionischen amphiphilen Lipide, gegebenenfalls in Assoziation mit einem Stabilisierungsmittel enthält, wobei die Vesikeln in einer kontinuierlichen wäßrigen Phase dispergiert sind, welche enthält:
1) wenigstens ein kationisches grenzflächenaktives Mitel der Formel 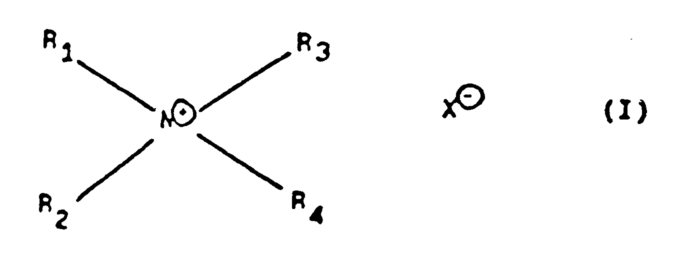 worin X für Chlor oder CH₃SO₄⁻ steht und R₁ einen C₁-C₄-Alkylrest bedeutet und worin:
a) wenn X für Chlor steht:
- R₂ und R₃ C₁-C₄-Alkylreste bedeuten, die untereinander gleich oder verschieden oder zu R₁ gleich oder verschieden sind, und R₄ einen C₁₆-C₂₂-Alkylrest bedeutet;
- oder R₂ = R₁, wobei in diesem Fall:
R₃ = R₄ = C₁₈-Alkyl; oder
R₃ = (C₁₇-Alkyl)amidopropyl und
R₄ = (C₁₄-Alkyl)acetat und
b) wenn X für CH₃SO₄⁻ steht:
- R₂ einen (Alkyl- und/oder Alkenyl)amidoethylrest bedeutet, wobei der Alkyl- und/oder Alkenylrest ein C₁₃-C₂₁-Rest ist und von Talgfettsäuren abgeleitet ist;
- R₃ und R₄ zusammen mit dem Stickstoffatom einen in 2-Position substituierten 4,5-Dihydroimidazolring bilden; und/oder
2) wenigstens ein quaternisiertes Protein, das ein chemisch modifiziertes Polypeptid darstellt, welches am Ende der Kette oder aufgepfropft wenigstens eine quaternäre Ammoniumgruppe aufweist, welche wenigstens eine C₁-C₁₈-Alkylkette umfaßt, wobei das Polypeptid ausgewählt ist unter Hydrolysaten tierischer Proteine;
unter der Voraussetzung, daß die Zusammensetzung zwingend wenigstens ein wie oben unter 2) definiertes Protein enthält, wenn die Zusammensetzung Distearyidimethylammoniumchlorid als kationisches grenzflächenaktives Mittel enthält,
wobei das kationische grenzflächenaktive Mittel (die kationischen grenzflächenaktiven Mittel), wenn es (sie) vorhanden ist (sind), 0,05 bis 10 Gew.-% des gesamten Mittels ausmacht (ausmachen), das erwähnte quaternisierte Protein (die erwähnten quaternisierten Proteine), wenn es (sie) vorhanden ist (sind), 0,05 bis 3 Gew.-% des gesamten Mittels ausmacht (ausmachen) und das amphiphile Lipid (die amphiphilen Lipide), das (die) die lamellare Phase bildet (bilden), 0,1 bis 20 Gew.-% des gesamten Mittels ausmacht (ausmachen).

2. Mittel nach Anspruch 1, dadurch gekennzeichnet, daß die Vesikel einen mittleren Durchmesser im Bereich von 0,01 bis 5 µm, vorzugsweise 0,05 bis 0,35 µm, aufweisen.

3. Mittel nach einem der Ansprüche 1 oder 2, dadurch gekennzeichnet, daß das ionische amphiphile Lipid (die ionischen amphiphilen Lipide) ausgewählt ist (sind) unter natürlichen oder synthetischen Phospholipiden.

4. Mittel nach einem der Ansprüche 1 oder 2, dadurch gekennzeichnet, daß das ionische amphiphile Lipid (die ionischen amphiphilen Lipide) ausgewählt ist (sind) unter amphoteren Verbindungen, die zwei lipophile Ketten oder eine Assoziation von zwei langkettigen organischen Ionen mit entgegengesetzter Ladung umfassen.

5. Mittel nach einem der Ansprüche 1 oder 2, dadurch gekennzeichnet, daß das ionische amphiphile Lipid (die ionischen amphiphilen Lipide) ausgewählt ist (sind) unter anionischen Verbindungen.

6. Mittel nach Anspruch 5, dadurch gekennzeichnet, daß das ionischen amphiphile Lipid (die ionischen amphiphilen Lipide) ausgewählt ist (sind) unter Verbindungen der Formel: 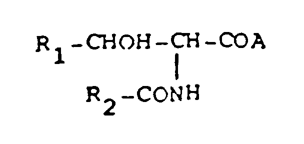 worin:
- R¹ einen C₇-C₂₁-Alkyl- oder -Alkenylrest bedeutet;
- R² einen gesättigten oder ungesättigten C₇-C₃₁-Kohlenwasserstoffrest bedeutet;
- COA für eine Gruppe steht, die ausgewählt ist unter folgenden Gruppen:
- COOM, wobei M für H, Na, K, NH₄ oder ein von einem Amin abgeleitetes substituiertes Ammoniumion steht;
- einem Rest 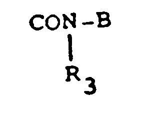 wobei B für einen von primären oder sekundären, mono- oder polyhydroxylierten Aminen abgeleiteten Rest steht und R₃ ein Wasserstoffatom oder einen Methyl-, Ethyl- oder Hydroxyethylrest bedeutet;
- einem Rest 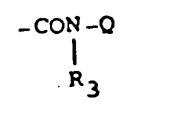 worin Q einen substituierten Aminoalkyl- oder Ammonioalkylrest bedeutet und R₃ die oben angegebenen Bedeutungen besitzt; und
- COOZ, wobei Z für einen C₃-C₇-Polyolrest steht.

7. Mittel nach einem der Ansprüche 1 bis 6, dadurch gekennzeichnet, daß das ionische Lipid (die ionischen Lipide) das (die) die hydratisierte lamellare Lipidphase bildet (bilden), mit wenigstens einem Stabilisierungsmittel assoziiert ist (sind), das ausgewählt ist unter Sterolen, Mono- oder Dinatriumsalzen von Acylglutamaten, einem C₁₄-C₂₂-Acylrest, Phosphorsäureestern von C₁₂-C₁₆-Fettaikoholen und oxyethylenierten Phytosterolen.

8. Mittel nach einem der Ansprüche 1 bis 7, dadurch gekennzeichnet, daß das ionische amphiphile Lipid (die ionischen amphiphilen Lipide), das (die) die hydratisierte lamellare Lipidphase bildet (bilden), assoziiert ist (sind) mit wenigstens einem anionischen Stabilisierungsmittel in einer Menge von höchstens gleich 12 Gew.-% und/oder mit wenigstens einem Sterol in einer Menge von höchstens gleich 100 Gew.-%, wobei der Anteil in beiden Fällen, bezogen auf das Gewicht des ionischen amphiphilen Lipids (oder der ionischen amphiphilen Lipide), berechnet ist.

9. Mittel nach einem der Ansprüche 1 bis 8, dadurch gekennzeichnet, daß die hydratisierte lamellare Lipidphase Wasser gegebenenfalls in Anwesenheit eines kosmetisch oder eines pharmazeutisch wirksamen Mittels enthält.

10. Mittel nach Anspruch 9, bei dem die hydratisierte lamellare Lipidphase einen Wirkstoff enthält, dadurch gekennzeichnet, daß der Wirkstoff ausgewählt ist unter Mitteln gegen Haarausfall oder für das Nachwachsen der Haare, Retinaiden und verwandten Verbindungen, antiinflammatorischen Mitteln, antifungischen Mitteln, antiseborrhoischen Mitteln und Sonnenfiltern.

11. Mittel nach einem der Ansprüche 1 bis 10, dadurch gekennzeichnet, daß das in der kontinuierlichen wäßrigen Phase enthaltene kationische grenzflächenaktive Mittel ausgewählt ist unter:
a) Tetraalkylammoniumhalogeniden;
b) Stearamidopropyl-dimethyl(myristylacetat)ammoniumchlorid der Formel: 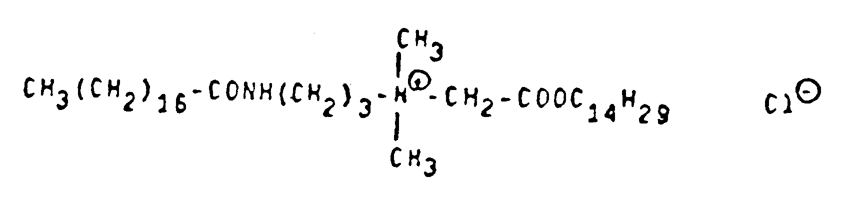
c) einem quaternären Ammoniumsalz der Formel: 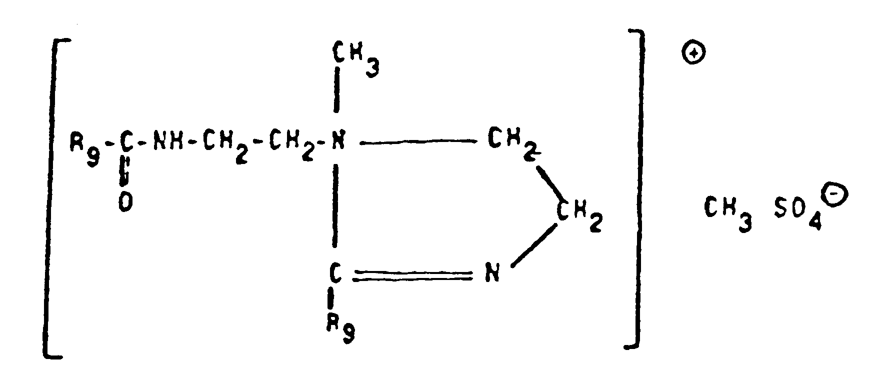 worin R₉ für ein von Talgfettsäuren abgeleitetes Gemisch von C₁₃-C₂₁-Alkenyl- und/oder -Alkylresten steht.

12. Mittel nach einem der Ansprüche 1 bis 11, dadurch gekennzeichnet, daß das quaternisierte Protein ausgewählt ist unter:
a) Hydrolysaten tierischer Proteine, die auf der Polypeptidkette quaternäre Ammoniumgruppen aufweisen, welche wenigstens einen C₁-C₁₈-Alkylrest umfassen;
b) Hydrolysaten tierischer Proteine, die Trimethylbenzylammoniumgruppen tragen;
c) Collagenhydrolysaten, die Triethylammoniumgruppen tragen;
d) Collagenhydrolysaten, die Trimethylammonium- und Trimethylstearylammoniumgruppen tragen;
e) einem quaternisierten Protein, das sich aus der Kondensation von Cocoamidopropyl-dimethylamin mit einem hydrolysierten tierischen Protein ergibt.

13. Mittel nach einem der Ansprüche 1 bis 12, dadurch gekennzeichnet, daß es gleichzeitig wenigstens ein kationisches grenzflächenaktives Mittel und wenigstens ein quaternisiertes Protein enthält.

14. Mittel nach Anspruch 13, dadurch gekennzeichnet, daß es einerseits Behenyltrimethylammoniumchlorid und andererseits ein tierisches Proteinhydrolysat enthält, welches auf der Polypeptidkette quaternisierte Ammoniumgruppen aufweist, die wenigstens eine C₁-C₁₈-Alkylkette aufweisen, wobei die Polypeptidkette ein mittleres Molekulargewicht von etwa 12000 aufweist.

15. Mittel nach einem der Ansprüche 1 bis 14, dadurch gekennzeichnet, daß die kontinuierliche wäßrige Phase wenigstens ein Additiv enthält, das ausgewählt ist unter Konservierungsmitteln, Stabilisierungsmitteln, Farbstoffen, Feuchthaltemitteln, weichmachenden Mitteln, Parfüms und Verdickungsmitteln.

16. Mittel nach einem der Ansprüche 1 bis 15, das wenigstens ein kationisches grenzflächenaktives Mittel enthält, dadurch gekennzeichnet, daß das kationiche grenzflächenaktive Mittel 0,1 bis 6 Gew.-% des Gesamtgewichtes des Mittels ausmacht.

17. Mittel nach einem der Ansprüche 1 bis 16, das wenigstens ein quaternisiertes Protein enthält, dadurch gekennzeichnet, daß das quaternisierte Protein 0,05 bis 0,5 Gew.-% des Gesamtgeewichts des Mittels ausmacht.

18. Mittel nach einem der Ansprüche 1 bis 17, dadurch gekennzeichnet, daß das ionische amphiphile Lipid (die ionischen amphiphilen Lipide), das (die) die lamellare Phase bildet (bilden, 1 bis 10 Gew.-% und insbesondere 3 bis 10 Gew.-% des Gesamtgewichts des Mittels ausmacht (ausmachen).

19. Verfahren zur Behandlung der Haare und der Kopfhaut, dadurch gekennzeichnet, daß man auf den Kopf 20 bis 40 g eines kosmetischen Mittels nach einem der Ansprüche 1 bis 18 aufträgt, 1 bis 15 Minuten in Kontakt läßt und anschließend mit Wasser spült.

20. Verfahren zur Behandlung der Haare und der Kopfhaut, dadurch gekennzeichnet, daß man auf den Kopf 5 bis 10 g eines kosmetischen Mittels nach einem der Ansprüche 1 bis 18 aufträgt.

## Patentansprüche (Patentansprüche für folgende(n) Vertragsstaat(en): ES, GR)

1. Kosmetisches Mittel zur Behandlung der Haare und der Kopfhaut, das Vesikeln enthält welche von einem hydratisierten in Wasser unlöslichen lamellaren Lipidphase gebildet sind, wobei die Lipidphase ionischen amphiphilen Lipide, gegebenenfalls in Assoziation mit einem Stabilisierungsmittel enthält, wobei die Vesikeln in einer kontinuierlichen wäßrigen Phase dispergiert sind, welche enthält:
1) wenigstens ein kationisches grenzflächenaktives Mitel der Formel 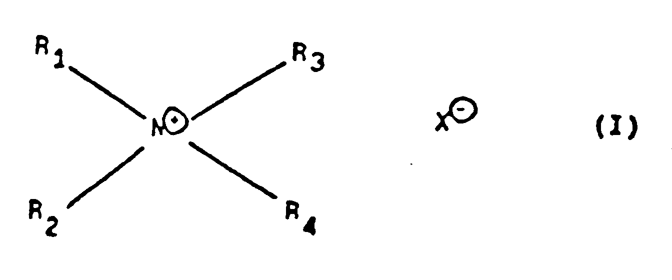 worin X für Chlor oder CH₃SO₄⁻ steht und R₁ einen C₁-C₄-Alkylrest bedeutet und worin:
a) wenn X für Chlor steht:
- R₂ und R₃ C₁-C₄-Alkylreste bedeuten, die untereinander gleich oder verschieden oder zu R₁ gleich oder verschieden sind, und R₄ einen C₁₆-C₂₂-Alkylrest bedeutet;
- oder R₂ = R₁, wobei in diesem Fall:
R₃ = R₄ = C₁₈-Alkyl; oder
R₃ = (C₁₇-Alkyl)amidopropyl und
R₄ = (C₁₄-Alkyl)acetat und
b) wenn X für CH₃SO₄⁻ steht:
- R₂ einen (Alkyl- und/oder Alkenyl)amidoethylrest bedeutet, wobei der Alkyl- und/oder Alkenylrest ein C₁₃-C₂₁-Rest ist und von Talgfettsäuren abgeleitet ist;
- R₃ und R₄ zusammen mit dem Stickstoffatom einen in 2-Position substituierten 4,5-Dihydroimidazolring bilden; und/oder
2) wenigstens ein quaternisiertes Protein, das ein chemisch modifiziertes Polypeptid darstellt, welches am Ende der Kette oder aufgepfropft wenigstens eine quaternäre Ammoniumgruppe aufweist, welche wenigstens eine C₁-C₁₈-Alkylkette umfaßt, wobei das Polypeptid ausgewählt ist unter Hydrolysaten tierischer Proteine;
unter der Voraussetzung, daß die Zusammensetzung zwingend wenigstens ein wie oben unter 2) definiertes Protein enthält, wenn die Zusammensetzung Distearyldimethylammoniumchlorid als kationisches grenzflächenaktives Mittel enthält,
wobei das kationische grenzflächenaktive Mittel (die kationischen grenzflächenaktiven Mittel), wenn es (sie) vorhanden ist (sind), 0,05 bis 10 Gew.-% des gesamten Mittels ausmacht (ausmachen), das erwähnte quaternisierte Protein (die erwähnten quaternisierten Proteine), wenn es (sie) vorhanden ist (sind), 0,05 bis 3 Gew.-% des gesamten Mittels ausmacht (ausmachen) und das amphiphile Lipid (die amphiphilen Lipide), das (die) die lamellare Phase bildet (bilden), 0,1 bis 20 Gew.-% des gesamten Mittels ausmacht (ausmachen).

2. Mittel nach Anspruch 1, dadurch gekennzeichnet, daß die Vesikel einen mittleren Durchmesser im Bereich von 0,01 bis 5 µm, vorzugsweise 0,05 bis 0,35 µm, aufweisen.

3. Mittel nach einem der Ansprüche 1 oder 2, dadurch gekennzeichnet, daß das ionische amphiphile Lipid (die ionischen amphiphilen Lipide) ausgewählt ist (sind) unter natürlichen oder synthetischen Phospholipiden.

4. Mittel nach einem der Ansprüche 1 oder 2, dadurch gekennzeichnet, daß das ionische amphiphile Lipid (die ionischen amphiphilen Lipide) ausgewählt ist (sind) unter amphoteren Verbindungen, die zwei lipophile Ketten oder eine Assoziation von zwei langkettigen organischen Ionen mit entgegengesetzter Ladung umfassen.

5. Mittel nach einem der Ansprüche 1 oder 2, dadurch gekennzeichnet, daß das ionische amphiphile Lipid (die ionischen amphiphilen Lipide) ausgewählt ist (sind) unter anionischen Verbindungen.

6. Mittel nach Anspruch 5, dadurch gekennzeichnet, daß das ionischen amphiphile Lipid (die ionischen amphiphilen Lipide) ausgewählt ist (sind) unter Verbindungen der Formel: 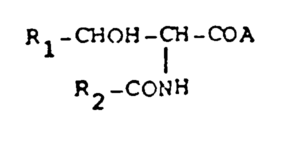 worin:
- R¹ einen C₇-C₂₁-Alkyl- oder -Alkenylrest bedeutet;
- R² einen gesättigten oder ungesättigten C₇-C₃₁-Kohlenwasserstoffrest bedeutet;
- COA für eine Gruppe steht, die ausgewählt ist unter folgenden Gruppen:
- COOM, wobei M für H, Na, K, NH₄ oder ein von einem Amin abgeleitetes substituiertes Ammoniumion steht;
- einem Rest 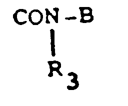 wobei B für einen von primären oder sekundären, mono- oder polyhydroxylierten Aminen abgeleiteten Rest steht und R₃ ein Wasserstoffatom oder einen Methyl-, Ethyl- oder Hydroxyethylrest bedeutet;
- einem Rest 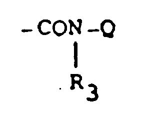 worin Q einen substituierten Aminoalkyl- oder Ammonioalkylrest bedeutet und R₃ die oben angegebenen Bedeutungen besitzt; und
- COOZ, wobei Z für einen C₃-C₇-Polyolrest steht.

7. Mittel nach einem der Ansprüche 1 bis 6, dadurch gekennzeichnet, daß das ionische Lipid (die ionischen Lipide) das (die) die hydratisierte lamellare Lipidphase bildet (bilden), mit wenigstens einem Stabilisierungsmittel assoziiert ist (sind), das ausgewählt ist unter Sterolen, Mono- oder Dinatriumsalzen von Acylglutamaten, einem C₁₄-C₂₂-Acylrest, Phosphorsäureestern von C₁₂-C₁₆-Fettalkoholen und oxyethylenierten Phytosterolen.

8. Mittel nach einem der Ansprüche 1 bis 7, dadurch gekennzeichnet, daß das ionische amphiphile Lipid (die ionischen amphiphilen Lipide), das (die) die hydratisierte lamellare Lipidphase bildet (bilden), assoziiert ist (sind) mit wenigstens einem anionischen Stabilisierungsmittel in einer Menge von höchstens gleich 12 Gew.-% und/oder mit wenigstens einem Sterol in einer Menge von höchstens gleich 100 Gew.-%, wobei der Anteil in beiden Fällen, bezogen auf das Gewicht des ionischen amphiphilen Lipids (oder der ionischen amphiphilen Lipide), berechnet ist.

9. Mittel nach einem der Ansprüche 1 bis 8, dadurch gekennzeichnet, daß die hydratisierte lamellare Lipidphase Wasser gegebenenfalls in Anwesenheit eines kosmetisch aktiven Mittels enthält.

10. Mittel nach Anspruch 9, bei dem die hydratisierte lamellare Lipidphase einen Wirkstoff enthält, dadurch gekennzeichnet, daß der Wirkstoff ausgewählt ist unter Mitteln gegen Haarausfall oder für das Nachwachsen der Haare, Retinoiden und verwandten Verbindungen, antiinflammatorischen Mitteln, antifungischen Mitteln, antiseborrhoischen Mitteln und Sonnenfiltern.

11. Mittel nach einem der Ansprüche 1 bis 10, dadurch gekennzeichnet, daß das in der kontinuierlichen wäßrigen Phase enthaltene kationische grenzflächenaktive Mittel ausgewählt ist unter:
a) Tetraalkylammoniumbalogeniden;
b) Stearamidopropyl-dimethyl(myristylacetat)ammoniumchlorid der Formel: 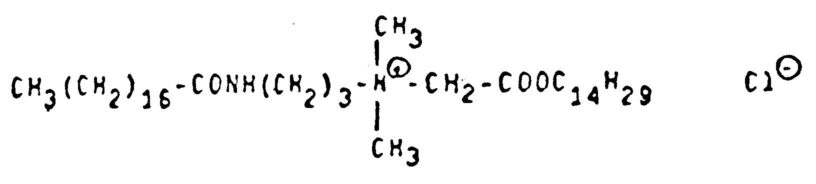
c) einem quaternären Ammoniumsalz der Formel: 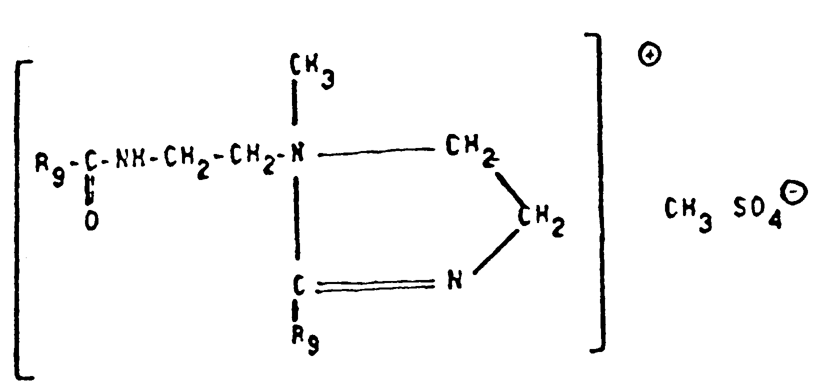 worin R₉ für ein von Talgfettsäuren abgeleitetes Gemisch von C₁₃-C₂₁-Alkenyl- und/oder -Alkylresten steht.

12. Mittel nach einem der Ansprüche 1 bis 11, dadurch gekennzeichnet, daß das quaternisierte Protein ausgewählt ist unter:
a) Hydrolysaten tierischer Proteine, die auf der Polypeptidkette quaternäre Ammoniumgruppen aufweisen, welche wenigstens einen C₁-C₁₈-Alkylrest umfassen;
b) Hydrolysaten tierischer Proteine, die Trimethylbenzylammoniumgruppen tragen;
c) Collagenhydrolysaten, die Triethylammoniumgruppen tragen;
d) Collagenhydrolysaten, die Trimethylammonium- und Trimethylstearylammoniumgruppen tragen;
e) einem quaternisierten Protein, das sich aus der Kondensation von Cocoamidopropyl-dimethylamin mit einem hydrolysierten tierischen Protein ergibt.

13. Mittel nach einem der Ansprüche 1 bis 12, dadurch gekennzeichnet, daß es gleichzeitig wenigstens ein kationisches grenzflächenaktives Mittel und wenigstens ein quaternisiertes Protein enthält.

14. Mittel nach Anspruch 13, dadurch gekennzeichnet, daß es einerseits Behenyltrimethylammoniumchlorid und andererseits ein tierisches Proteinhydrolysat enthält, welches auf der Polypeptidkette quaternisierte Ammoniumgruppen aufweist, die wenigstens eine C₁-C₁₈-Alkylkette aufweisen, wobei die Polypeptidkette ein mittleres Molekulargewicht von etwa 12000 aufweist.

15. Mittel nach einem der Ansprüche 1 bis 14, dadurch gekennzeichnet, daß die kontinuierliche wäßrige Phase wenigstens ein Additiv enthält, das ausgewählt ist unter Konservierungsmitteln, Stabilisierungsmitteln, Farbstoffen, Feuchthaltemitteln, weichmachenden Mitteln, Parfüms und Verdickungsmitteln.

16. Mittel nach einem der Ansprüche 1 bis 15, das wenigstens ein kationisches grenzflächenaktives Mittel enthält, dadurch gekennzeichnet, daß das kationiche grenzflächenaktive Mittel 0,1 bis 6 Gew.-% des Gesamtgewichtes des Mittels ausmacht.

17. Mittel nach einem der Ansprüche 1 bis 16, das wenigstens ein quaternisiertes Protein enthält, dadurch gekennzeichnet, daß das quaternisierte Protein 0,05 bis 0,5 Gew.-% des Cesamtgeewichts des Mittels ausmacht.

18. Mittel nach einem der Ansprüche 1 bis 17, dadurch gekennzeichnet, daß das ionische amphiphile Lipid (die ionischen amphiphilen Lipide), das (die) die lamellare Phase bildet (bilden, 1 bis 10 Gew.-% und insbesondere 3 bis 10 Gew.-% des Gesamtgewichts des Mittels ausmacht (ausmachen).

19. Verfahren zur Behandlung der Haare und der Kopfhaut, dadurch gekennzeichnet, daß man auf den Kopf 20 bis 40 g eines kosmetischen Mittels nach einem der Ansprüche 1 bis 18 aufträgt, 1 bis 15 Minuten in Kontakt läßt und anschließend mit Wasser spült.

20. Verfahren zur Behandlung der Haare und der Kopfhaut, dadurch gekennzeichnet, daß man auf den Kopf 5 bis 10 g eines kosmetischen Mittels nach einem der Ansprüche 1 bis 18 aufträgt.
